# EUROPÄISCHE PATENTSCHRIFT

(11) **EP 2 708 199 B1**
(45) Veröffentlichungstag und Bekanntmachung des Hinweises auf die Patenterteilung: **22.11.2017**
(21) Anmeldenummer: 13184978.8
(22) Anmeldetag: 18.09.2013
(51) Int. Cl.: A61B 17/80, A61B 90/00

(54) **Verschließteil für einen Brustbeinverschluss, Brustbeinverschlusssystem mit einem solchen Verschließteil sowie Brustbeinverschluss**
Sternum closure in the form of a sternal plate with an integral separation component
Fermeture du sternum selon la méthode d'une plaque sternale avec composant de séparation intégral

(30) Priorität: 18.09.2012 EP 12184854
(43) Veröffentlichungstag der Anmeldung: 19.03.2014
(73) Patentinhaber: Karl Leibinger Medizintechnik GmbH & Co. KG, 78570 Mühlheim/Donau (DE)
(72) Erfinder: Waizenegger, Axel, 78570 Muehlheim (DE); Koett, Thomas, 78600 Kolbingen (DE)
(74) Vertreter: Winter, Brandl, Fürniss, Hübner, Röss, Kaiser, Polte - Partnerschaft mbB

(56) Entgegenhaltungen:
- WO-A1-03/061493
- WO-A1-2012/162733
- US-A1- 2002 128 654
- US-A1- 2011 125 193

## Beschreibung

Die Erfindung betrifft ein Verschließteil für einen Brustbeinverschluss nach dem Oberbegriff des Anspruchs 1, welcher Brustbeinverschluss an einem menschlichen oder tierischen Körper im Bereich des Brustbeins zum Verschließen eines Spaltes darin anbringbar ist, sowie ein Brustbeinverschlusssystem, umfassend zwei voneinander beabstandete und am menschlichen oder tierischen Körper beiderseits eines Spaltes im Bereich eines Brustbeins anbringbare erste und zweite Befestigungsabschnitte sowie ein solches Verschließteil. Das Verschließteil weist zudem einen ersten Hintergriffbereich und einen zweiten Hintergriffbereich auf, wobei zum einen der erste Hintergriffbereich zum formschlüssigen Befestigen an einem ersten Anbringbereich (auch als erster Knochenplattenbereich bezeichenbar) des Brustbeinverschlusses auf der einen Seite des Spalts vorbereitet ist (d.h. angeordnet, bemessen und ausgerichtet ist) und zum anderen der zweite Hintergriffbereich zum formschlüssigen Befestigen an einem zweiten Anbringbereich (auch als zweiter Knochenplattenbereich bezeichenbar) des Brustbeinverschlusses auf der anderen Seite des Spalts vorbereitet ist (d.h. angeordnet, bemessen und ausgerichtet ist). Ein als erste Verschließteilhälfte ausgebildeter Bestandteil und ein als zweite Verschließteilhälfte ausgebildeter Bestandteil sind miteinander lösbar verbunden. Konventionelle Verschließteile, Brustbeinverschlüsse und Brustbeinverschlusssysteme mit diesen Bestandteilen sind bereits aus dem Stand der Technik bekannt. Beispielsweise sind mehrere, unterschiedlich ausgeführte Brustbeinverschlüsse aus der US 2002/0128654 A1 bekannt, wobei konkret eine Vorrichtung für eine Knochenverbindung über einer Fraktur oder Osteotomie, offenbart ist. Diese Vorrichtung umfasst eine Platte mit einer oberen Oberfläche und einer unteren Oberfläche, und mit mindestens zwei Knochenfixierungsbereichen, und einem Brückenbereich zwischen den Knochenfixierungsbereichen, wobei jeder Knochenfixierungsbereich mindestens eine Öffnung aufweist und der Brückenbereich einen Spalt zwischen dem Brückenbereich und dem Knochen ausgestaltet um den Eingriff einer geeigneten Trennvorrichtung auf den Brückenbereich zu ermöglichen. Auch ist eine Befestigungseinrichtung umfasst, die angepasst ist, um durch die Öffnungen hindurch angeordnet zu werden und um in Eingriff mit der Platte zu gelangen, und die Knochenfixierungsbereiche an einem oder mehreren Abschnitten eines Knochens zu befestigen. Diese Verschlüsse haben jedoch allesamt den Nachteil, dass sie, wenn einmal getrennt, bspw. bei einer Notoperation, nicht einfach wieder im Bereich der Trennfuge zusammengefügt werden können. Für ein erneutes Verschließen des Brustbeins müsste dann entweder ein neuer Brustbeinverschluss, versetzt zu dem alten, durchtrennten

Brustbeinverschluss zusätzlich im Bereich des Brustbeins befestigt werden oder alternativ dazu zuerst der alte Brustbeinverschluss herausgenommen werden und dann erst der neue Brustbeinverschluss an der Stelle des Alten eingesetzt werden. Dadurch wird das Wiederverschließen/ Verschließen eines bei einer Notoperation nochmals durchtrennten Brustbeins wesentlich erschwert.

Die WO 2008/073898 A2 offenbart außerdem einen Sternumverschluss mit einer ersten Platte, mit einem Knochenfixierungsbereich, wobei der Knochenfixierungsbereich mindestens eine versenkte Öffnung zur Aufnahme eines Befestigungsmittels zum Befestigen der Platte an einer Seite des Brustbeins aufweist, und einem Verriegelungsbereich mit mindestens einem hervorstehenden Ausrichtelement, und einer zweiten Platte, mit einem Knochenfixierungsbereich, wobei der Knochenfixierungsbereich mindestens eine versenkten Öffnung zur Aufnahme eines Befestigungsmittels zum Befestigen der Platte an einer gegenüberliegenden Seiten des Brustbeins aufweist, und einem Verriegelungsbereich, der so dimensioniert ist, dass er zumindest einen Teil des Verriegelungsbereiches der ersten Platte kontaktiert. Zudem ist ein Drehelement vorhanden, das mit dem mindestens einen Ausrichtelement auf der ersten Platte, zum Zusammenhalten der ersten und zweiten Platten und somit die gegenüberliegenden Seiten des Brustbeins zu sichern, zusammenwirkt, wobei die ersten und zweiten Platten durch Auskuppeln des Drehelementes von dem mindestens einen Ausrichtelement getrennt werden können. Durch diesen wiederverschließbaren Brustbeinverschluss ist es zwar möglich bei einem medizinischen Notfall das zuvor verschlossene Brustbein rasch wieder zu öffnen, es hat sich jedoch dabei der Nachteil herausgestellt, dass es aufgrund der sich überlappenden Bereiche der Knochenfixierungsbereiche, oberhalb des Trennspaltes im Brustbein, zu Behinderungen bei dem medizinischen Eingriff kommen kann. Da die Knochenfixierungsbereiche unmittelbar den Spalt des getrennten Brustbeins verdecken, ist es, bei einem bereits schon wieder etwas zugewachsenen Brustbein, nicht möglich, ohne ein Zerstören des Verschlusses, das Brustbein mit einer Säge zu öffnen. Auch bei einem noch nicht ausreichend fest zusammengewachsenem Brustbein kann es zu Behinderungen kommen. Die Brustbeinhälften müssen entweder so weit auseinander gedrückt/ gespreizt werden, dass es zu Beeinträchtigungen des zu behandelnden Körpers durch die aufzuwendende Spreizkraft kommen kann, oder, alternativ dazu, muss der zu behandelnde Arzt seitlich dieser Knochenfixierungsbereiche herumoperieren, wodurch das Operationsverfahren behindert werden kann.

Weiterer Stand der Technik ist aus der WO2012/162733 A1 bekannt. Ein Verschließteil für einen Brustbeinverschluss mit den Merkmalen des Oberbegriffs des Anspruchs 1 ist aus der US 2011/125193 A1 bekannt. Es ist somit Aufgabe der vorliegenden Erfindung ein Wiederverschließen eines Brustbeinverschlusses zu ermöglichen, bei dem eine Beeinträchtigung des Operationsprozesses vermieden werden soll.

Diese Aufgabe wird erfindungsgemäß durch die Merkmale des Anspruchs 1 gelöst.

Dadurch kann ein nach einer Notoperation nochmals geöffnetes, zuvor bereits zumindest schon einmal verschlossenes Brustbein auf einfache Weise wiederverschlossen werden, wodurch sich die Verschlusszeit bei einer Operation wesentlich verkürzt. Zugleich wird durch die zwei voneinander beabstandeten Hintergriffbereiche ein zuvor freigelegter Spalt im Brustbein überbrückt und die getrennten Brustbeinverschlusshälften ausreichend fest miteinander verbunden, wobei jeder Hintergriffbereich eine Hälfte des Brustbeinverschlusses am Anbringbereich festhält. Dadurch ist auch nach dem Wiederverschließen ein ausreichend starker Zusammenhalt des Brustbeins für die folgende Heilung zur Verfügung gestellt, wobei insbesondere der Verbund zwischen Knochen, Brustbein und den Anbringbereiche/ Knochenplattenbereichen verstärkt wird. Auch wird dadurch die Handhabung beim Wiederverschließen weiter vereinfacht, da das Verschließteil auf einmal auf die Anbringbereiche aufgesetzt / aufgesteckt werden kann. Eine besonders schnelle Fixierung des Verschließteils an den Anbringbereichen ist möglich. Die Zeit des Wiederverschließens des Brustbeins wird daher weiter reduziert. Zudem ist eine mehrteilige, etwa zweiteilige Struktur des Verschließteils besonders effektiv ausgestaltbar, da diese beiden Teile unmittelbar nebeneinander angeordnet werden können und unmittelbar ein Teil der Verrasterung oder Verschraubung ausbilden können. Die Bauteilkomplexität wird somit weiter verringert und die Anzahl der nötigen Bauteile reduziert.

Weitere vorteilhafte Ausführungsformen sind in den Unteransprüchen beansprucht und werden nachfolgend näher erläutert.

Ist das Verschließteil zweigeteilt ausgestaltet, so ist eine noch stabilere Verbindung zwischen den getrennten Brustbeinverschlusshälften möglich. Bei einer geteilten Ausgestaltung, z.B. einer Teilung in Längsrichtung (Teilungsebene verläuft in Längsrichtung) oder einer Teilung in Querrichtung (Teilungsebene verläuft quer/schräg oder im Wesentlichen senkrecht zur Längsrichtung), können die Hintergriffbereiche des Verschließteils zunächst einfach um sich verjüngende Bereiche/ Verjüngungsbereiche der Anbringbereiche/ Knochenplattenbereiche eingeschoben werden und somit zunächst in einem unverspannten Zustand zueinander an den Anbringbereichen des Brustbeinverschlusses angeordnet werden. Erst im Anschluss daran, durch ein Aufeinanderzubewegen der einzelnen Teile des Verschließteils werden die Anbringbereiche fest formschlüssig in dem Verschließteil eingespannt und relativ zueinander leicht gespannt, sodass die Brustbeinhälften über die Verbindung mit den Anbringbereichen möglichst spaltfrei aneinander anliegen und zueinander verspannt/ gegeneinander gedrückt sind.

Wenn das Verschließteil stofflich vom Brustbeinverschluss getrennt ist, kann das Verschließteil während des medizinischen Eingriffs am zu behandelnden Körper von dem Brustbeinverschluss abgenommen/ entfernt sein. Ein Mittelstück, das durch das Verschließteil ausgebildet ist, kann daher einfach entnommen werden. Die Behandlung wird dadurch weiter vereinfacht.

Gemäß einer weiteren Ausführungsform ist es auch von Vorteil, wenn der erste und/oder der zweite Hintergriffbereich als Eindringelement, etwa als Zapfen, Schraube, Haken, Bolzen oder Dorn, ausgebildet ist und/oder als Rippe, Nase, Vorsprung oder Grat ausgestaltet ist und/oder eine Führungsfläche ausformt. Die Führungsfläche ist an dem jeweiligen ersten und zweiten Hintergriffbereich vorzugsweise schalenförmige ausgestaltet. Dadurch ist eine besonders stabile, formschlüssige Anbindung der Anbringbereiche an dem Verschließteil umsetzbar. Die Eindringelemente, wie die Nasen, können dabei in Aussparungen in den jeweiligen Anbringbereichen unmittelbar eingeschoben werden und insbesondere während des Einschiebens in diesen geführt sein.

In diesem Zusammenhang ist es auch von Vorteil, wenn das Eindringelement integraler Bestandteil des Verschließteils ist oder als stofflich separates Bauteil ausgeführt ist und/oder das Eindringelement als Teil eines Schnappverschlusses ausgebildet ist. Dadurch ist eine noch direktere und stabilere Anbindung des Verschließteils an den Anbringbereichen möglich.

Ist der Schnappverschluss dabei, zusätzlich zu einem Formschluss an der gleichen oder einer anderen Stelle, eingesetzt, kann der Aufbau des Verschließteils weiter vereinfacht werden und/oder die Verschlusskraft durch das Verschließteil weiter erhöht werden, da Schnappverbindung eine hohe Haltekraft mit sich bringt.

Ist die Führungsfläche zudem quer/schräg zur Längsrichtung des Brustbeinverschlusses und/oder quer/schräg zur Längsrichtung des Spalts ausgerichtet, kann das Verschließteil sich bei einem leichten Verspannen der Hintergriffbereiche gegeneinander über die Führungsflächen zu den Anbringbereichen von selbst ausrichten und der Formschluss wirken.

Weiterhin ist auch ein Brustbeinverschlusssystem vorgesehen, das zwei voneinander beabstandete und am menschlichen oder tierischen Körper beiderseits eines Spaltes im Bereich eines Brustbeins anbringbare erste und zweite Befestigungsabschnitte sowie ein Verschließteil nach einem der oben genannten Ausführungsformen umfasst. Dadurch ist das gesamte Brustbeinverschlusssystem effizient herstellbar und eine wiederverschließbare Struktur kann bereits von vornherein, d. h. vor dem ersten Anbringen des Brustbeinverschlusssystems am Brustbein, zur Verfügung gestellt werden. Ein Wiederöffnen/ Widerverschließen kann daher bereits bei der ersten Operation umgesetzt werden. Eine Mehrteiligkeit ist daher von Vorteil.

Auch ist es in von Vorteil, wenn in einer Verschließstellung des Verschließteiles, zur festen Verbindung des ersten mit dem zweiten Befestigungsabschnitt, der erste Hintergriffbereich des Verschließteils formschlüssig in einem fest mit dem ersten Befestigungsabschnitt verbundenen ersten Anbringbereich eingreift und der zweite Hintergriffbereich des Verschließteils formschlüssig in einem fest mit dem zweiten Befestigungsabschnitt verbundenen zweiten Anbringbereich eingreift. Somit wird der Aufbau des Brustbeinverschlusssystems weiter vereinfacht.

Ferner ist ein Brustbeinverschluss zum Überbrücken eines Spaltes in einem menschlichen oder tierischen Brustbein vorgesehen, mit einem ersten Befestigungsabschnitt zum Anbringen auf der einen Seite des Spaltes und einem zweiten Befestigungsabschnitt zum Anbringen auf der anderen Seite des Spaltes, sowie mit einem den Spalt von dem ersten Befestigungsabschnitt zu dem zweiten Befestigungsabschnitt überbrückenden und stofflich mit diesem verbundenen Entnahmeteil, wobei das Entnahmeteil an zwei voneinander beabstandeten Durchtrennungsstellen trennbar ist, wobei das von beiden Befestigungsabschnitten entfernbare Entnahmeteil so bemessen ist, dass eine für einen Werkzeugeinsatz am Brustbein ausreichend große Arbeitsöffnung zwischen dem ersten Befestigungsabschnitt und dem zweiten Befestigungsabschnitt erreichbar ist. Damit ist auch ein Brustbeinverschluss besonders effizient herstellbar.

Auch ein Verfahren zum Verschließen eines Spaltes in einem menschlichen oder tierischen Brustbein mit einem Brustbeinverschluss ist hierin umfasst (nichterfindungsgemäß), bei welchem Verfahren der Brustbeinverschluss nach oben genannter Ausführung an einem Knochen- und/oder Knorpelgewebe angebracht (etwa angeschraubt) wird.

Weiterhin ist ein Verfahren zum Herstellen einer Arbeitsöffnung für eine Brustbeindurchtrennung oder eine Brustbeinöffnung an einem menschlichen oder tierischen Brustbein hierin enthalten (nichterfindungsgemäß), wobei ein an einem Knochen- und/oder Knorpelgewebe angebrachter Brustbeinverschluss nach oben genannter Ausführung an beiden Durchtrennungsstellen durchtrennt wird und gleichzeitig das Entnahmeteil von den Befestigungsabschnitten entfernt wird.

Ferner ist auch ein Verfahren zum Wiederverschließen eines Brustbeinverschlusses umfasst (nichterfindungsgemäß), wobei ein Verschließteil nach zumindest einer der oben genannten Ausführungen formschlüssig sowohl mit dem ersten Befestigungsabschnitt als auch mit dem zweiten Befestigungsabschnitt verbunden wird. Vorzugsweise wird das Verschließteil hierbei über den jeweiligen Anbringbereich mit dem jeweiligen Befestigungsabschnitt verbunden.

Die Erfindung wird nun nachfolgend mit Hilfe von Zeichnungen näher erläutert, in welchen unterschiedliche Ausführungsbeispiele dargestellt sind.

Es zeigen:

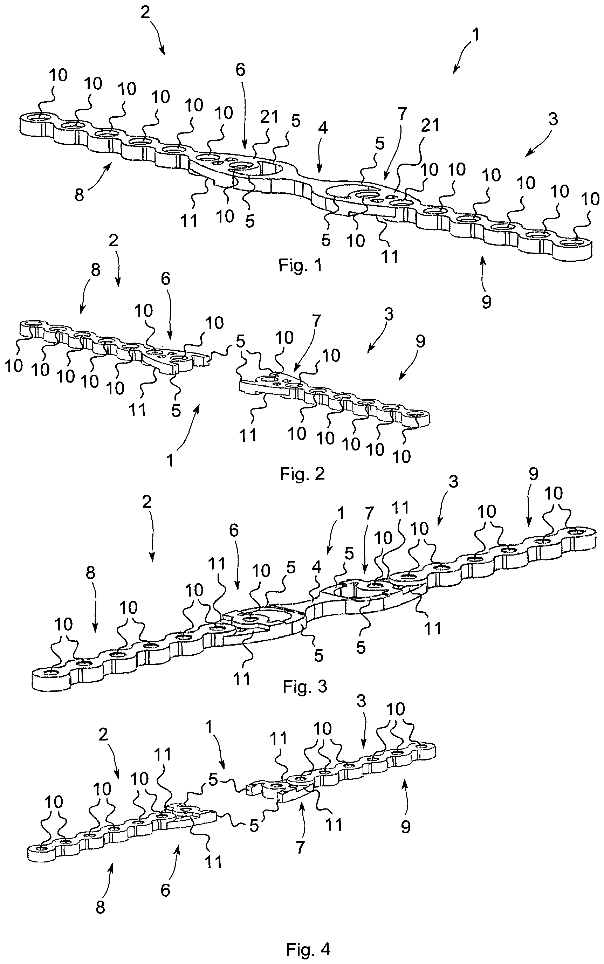

- Fig. 1: eine isometrische Darstellung eines Brustbeinverschlusses nach einer ersten Ausführungsform, wobei eine Vorderseite des Brustbeinverschlusses (d.h. eine dem Brustbein im befestigen Zustand zugewandte Seite) besonders gut zu erkennen ist und die beiden Befestigungsabschnitte des Brustbeinverschlusses über ein Entnahmeteil miteinander verbunden sind, und wobei der Brustbeinverschluss Aussparungen aufweist, in die ein Verschließteil formschlüssig eingreifen kann,
- Fig. 2: eine isometrische Darstellung der Vorderseite des in Fig. 1 dargestellten Brustbeinverschlusses, wobei der Brustbeinverschluss in einem Zustand befindlich ist, in dem das Entnahmeteil, nach dem Durchtrennen an mehreren Durchtrennungsstellen (zwei je Befestigungsabschnitt), entfernt/ entnommen ist,
- Fig. 3: eine isometrische Darstellung des in Fig. 1 dargestellten Brustbeinverschlusses, wobei eine Rückseite des Brustbeinverschlusses (d.h. eine im befestigen Zustand dem Brustbein abgewandte Seite) besonders gut zu erkennen ist und der Brustbeinverschluss wieder in dem Zustand befindlich ist, in dem das Entnahmeteil mit den Befestigungsabschnitten verbunden ist,
- Fig. 4: eine isometrische Darstellung der in Fig. 3 bereits dargestellten Rückseite des Brustbeinverschlusses, wobei der Brustbeinverschluss in einem Zustand befindlich ist, in dem das Entnahmeteil, nach dem Durchtrennen mehrerer Durchtrennungsstellen, entfernt/ entnommen ist,

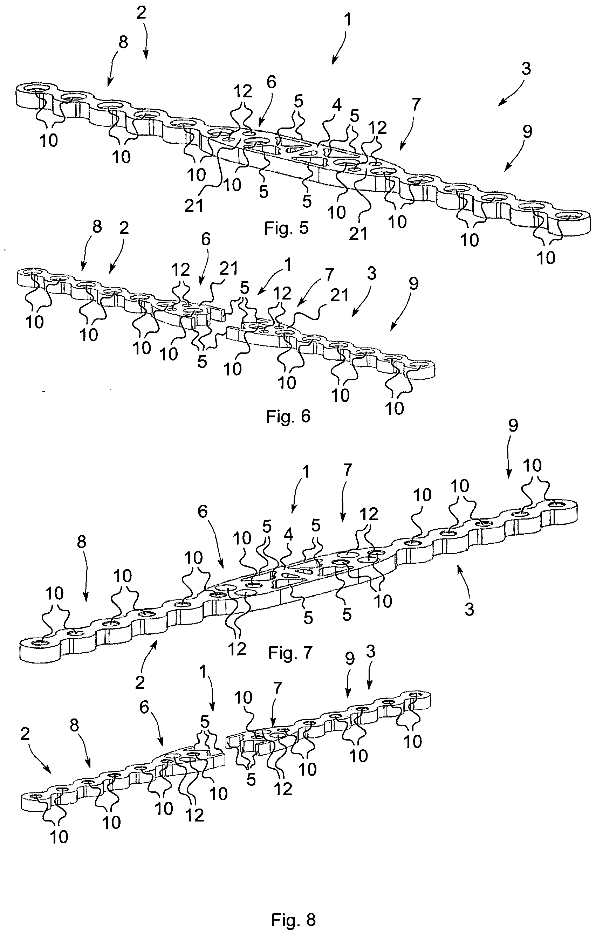

- Fig. 5: eine isometrische Darstellung eines Brustbeinverschlusses nach einer weiteren Ausführungsform, wobei eine Vorderseite des Brustbeinverschlusses (d.h. eine dem Brustbein im befestigen Zustand zugewandte Seite) besonders gut zu erkennen ist und die beiden Befestigungsabschnitte des Brustbeinverschlusses über ein Entnahmeteil miteinander verbunden sind, und wobei der Brustbeinverschluss Löcher aufweist, in die ein Schnappelement eines Verschließteils formschlüssig einsteckbar ist,
- Fig. 6: eine isometrische Darstellung der Vorderseite des in Fig. 5 dargestellten Brustbeinverschlusses, wobei der Brustbeinverschluss in einem Zustand befindlich ist, in dem das Entnahmeteil, nach dem Durchtrennen an mehreren Durchtrennungsstellen (drei je Befestigungsabschnitt), entfernt/ entnommen ist,
- Fig. 7: eine isometrische Darstellung des in Fig. 5 dargestellten Brustbeinverschlusses, wobei eine Rückseite des Brustbeinverschlusses (d.h. eine im befestigen Zustand dem Brustbein abgewandte Seite) besonders gut zu erkennen ist und der Brustbeinverschluss wieder in dem Zustand befindlich ist, in dem das Entnahmeteil mit den Befestigungsabschnitten verbunden ist,
- Fig. 8: eine isometrische Darstellung der in Fig. 7 bereits dargestellten Rückseite des Brustbeinverschlusses, wobei der Brustbeinverschluss in einem Zustand befindlich ist, in dem das Entnahmeteil, nach dem Durchtrennen an den Durchtrennungsstellen, entfernt/ entnommen ist,

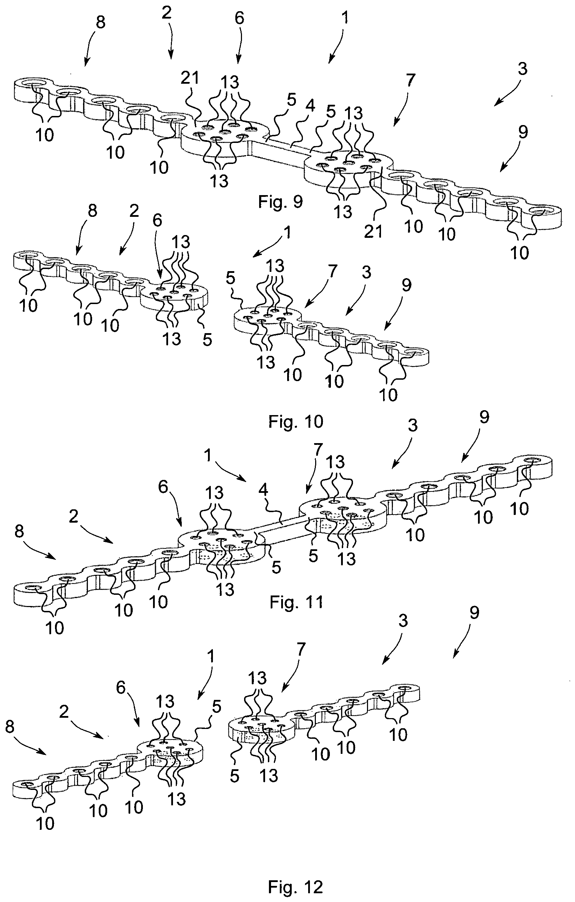

- Fig. 9: eine isometrische Darstellung eines Brustbeinverschlusses nach einer weiteren Ausführungsform, wobei eine Vorderseite des Brustbeinverschlusses (d.h. eine dem Brustbein im befestigen Zustand zugewandte Seite) besonders gut zu erkennen ist und die beiden Befestigungsabschnitte des Brustbeinverschlusses über ein Entnahmeteil miteinander verbunden sind, und wobei der Brustbeinverschluss Löcher aufweist, mit denen ein Befestigungsmittel eines Verschließteils fest verbindbar ist,
- Fig. 10: eine isometrische Darstellung der Vorderseite des in Fig. 9 dargestellten Brustbeinverschlusses, wobei der Brustbeinverschluss in einem Zustand befindlich ist, in dem das Entnahmeteil, nach dem Durchtrennen an mehreren Durchtrennungsstellen (eine je Befestigungsabschnitt), entfernt/ entnommen ist,
- Fig. 11: eine isometrische Darstellung des in Fig. 9 dargestellten Brustbeinverschlusses, wobei eine Rückseite des Brustbeinverschlusses (d.h. eine im befestigen Zustand dem Brustbein abgewandte Seite) besonders gut zu erkennen ist und der Brustbeinverschluss wieder in dem Zustand befindlich ist, in dem das Entnahmeteil mit den Befestigungsabschnitten verbunden ist,
- Fig. 12: eine isometrische Darstellung der in Fig. 11 bereits dargestellten Rückseite des Brustbeinverschlusses, wobei der Brustbeinverschluss in einem Zustand befindlich ist, in dem das Entnahmeteil, nach dem Durchtrennen an den Durchtrennungsstellen, entfernt/ entnommen ist,

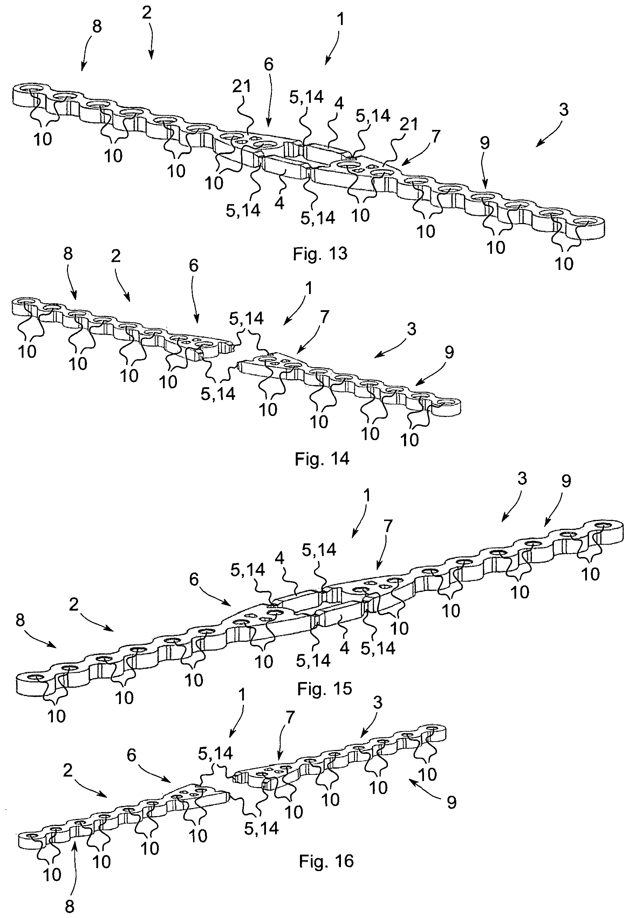

- Fig. 13: eine isometrische Darstellung eines Brustbeinverschlusses nach einer weiteren Ausführungsform, wobei eine Vorderseite des Brustbeinverschlusses (d.h. eine dem Brustbein im befestigen Zustand zugewandte Seite) besonders gut zu erkennen ist und die beiden Befestigungsabschnitte des Brustbeinverschlusses über ein Entnahmeteil miteinander verbunden sind, und wobei der Brustbeinverschluss Löcher aufweist, mit denen ein Befestigungsmittel eines Verschließteils fest verbindbar ist und die Durchtrennungsstellen durch einen sich verjüngenden Durchmesser des Entnahmeteils erkennbar sind,
- Fig. 14: eine isometrische Darstellung der Vorderseite des in Fig. 13 dargestellten Brustbeinverschlusses, wobei der Brustbeinverschluss in einem Zustand befindlich ist, in dem das Entnahmeteil, nach dem Durchtrennen an mehreren Durchtrennungsstellen (zwei je Befestigungsabschnitt), entfernt/ entnommen ist,
- Fig. 15: eine isometrische Darstellung des in Fig. 13 dargestellten Brustbeinverschlusses, wobei eine Rückseite des Brustbeinverschlusses (d.h. eine im befestigen Zustand dem Brustbein abgewandte Seite) besonders gut zu erkennen ist und der Brustbeinverschluss wieder in dem Zustand befindlich ist, in dem das Entnahmeteil mit den Befestigungsabschnitten verbunden ist,
- Fig. 16: eine isometrische Darstellung der in Fig. 15 bereits dargestellten Rückseite des Brustbeinverschlusses, wobei der Brustbeinverschluss in einem Zustand befindlich ist, in dem das Entnahmeteil, nach dem Durchtrennen an den Durchtrennungsstellen, entfernt/ entnommen ist,

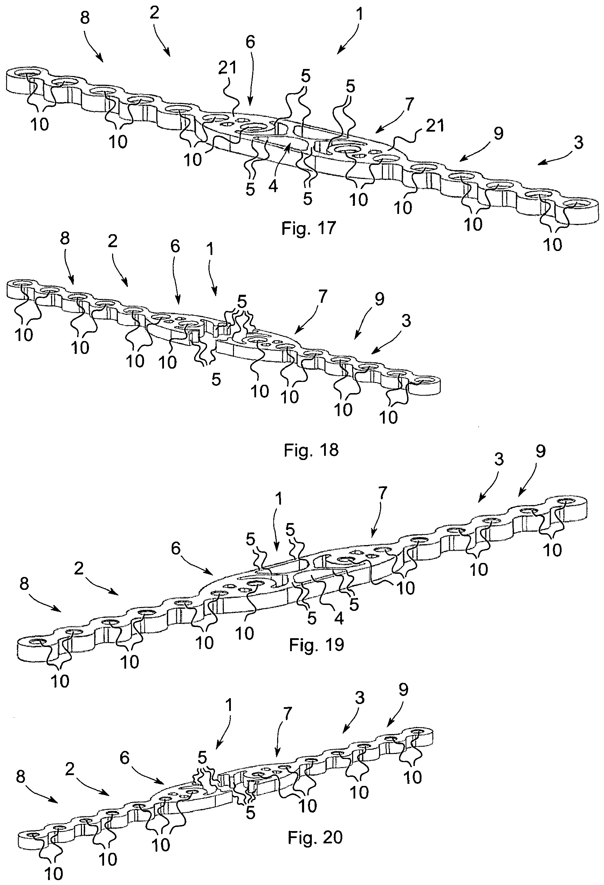

- Fig. 17: eine isometrische Darstellung eines Brustbeinverschlusses nach einer weiteren Ausführungsform, wobei eine Vorderseite des Brustbeinverschlusses (d.h. eine dem Brustbein im befestigen Zustand zugewandte Seite) besonders gut zu erkennen ist und die beiden Befestigungsabschnitte des Brustbeinverschlusses über ein Entnahmeteil miteinander verbunden sind, und wobei der Brustbeinverschluss Löcher aufweist, mit denen ein Befestigungsmittel eines Verschließteils fest verbindbar ist,
- Fig. 18: eine isometrische Darstellung der Vorderseite des in Fig. 17 dargestellten Brustbeinverschlusses, wobei der Brustbeinverschluss in einem Zustand befindlich ist, in dem das Entnahmeteil, nach dem Durchtrennen an mehreren Durchtrennungsstellen (vier je Befestigungsabschnitt), entfernt/ entnommen ist,
- Fig. 19: eine isometrische Darstellung des in Fig. 17 dargestellten Brustbeinverschlusses, wobei eine Rückseite des Brustbeinverschlusses (d.h. eine im befestigen Zustand dem Brustbein abgewandte Seite) besonders gut zu erkennen ist und der Brustbeinverschluss wieder in dem Zustand befindlich ist, in dem das Entnahmeteil mit den Befestigungsabschnitten verbunden ist,
- Fig. 20: eine isometrische Darstellung der in Fig. 19 bereits dargestellten Rückseite des Brustbeinverschlusses, wobei der Brustbeinverschluss in einem Zustand befindlich ist, in dem das Entnahmeteil, nach dem Durchtrennen an den Durchtrennungsstellen, entfernt/ entnommen ist,

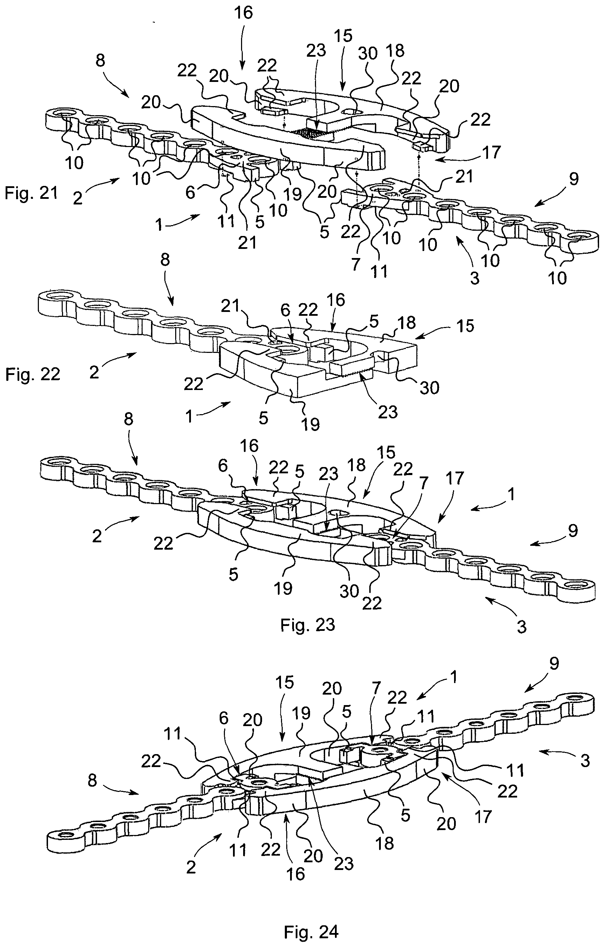

- Fig. 21: eine isometrische Darstellung eines erfindungsgemäßen Brustbeinverschluss-Verschließteil-Zusammenbaus in Explosionsansicht, worin der Brustbeinverschluss nach den Fig. 1 bis 4 eingesetzt ist und in dem Zustand befindlich ist, in dem das Entnahmeteil entfernt ist (Fig. 2 und 4), und mit einem zweiteiligen Verschließteil einer ersten Ausführungsform, welches Verschließteil mit Vorsprüngen versehen ist, die in die Aussparungen an Anbringbereichen der Befestigungsabschnitte einschiebbar sind,
- Fig. 22: eine isometrische Detailansicht einer Hälfte des Brustbeinverschluss-Verschließteil-Zusammenbaus nach Fig. 21, wobei das Verschließteil in einem an einem der Befestigungsabschnitte angebrachten Zustand dargestellt ist, in dem die Vorsprünge in die Aussparungen eingeschoben sind und die Teile des Verschließteils mittels integrierter Verrasterungen zueinander gesichert sind,
- Fig. 23: eine isometrische Darstellung des gesamten Brustbeinverschluss-Verschließteil-Zusammenbaus nach Fig. 21, wobei das Verschließteil in dem in Fig. 22 dargestellten Zustand befindlich ist, und die Vorderseite des Brustbeinverschlusses gezeigt ist,
- Fig. 24: eine isometrische Darstellung der Rückseite des gesamten Brustbeinverschluss-Verschließteil-Zusammenbaus nach Fig. 22 und 23,

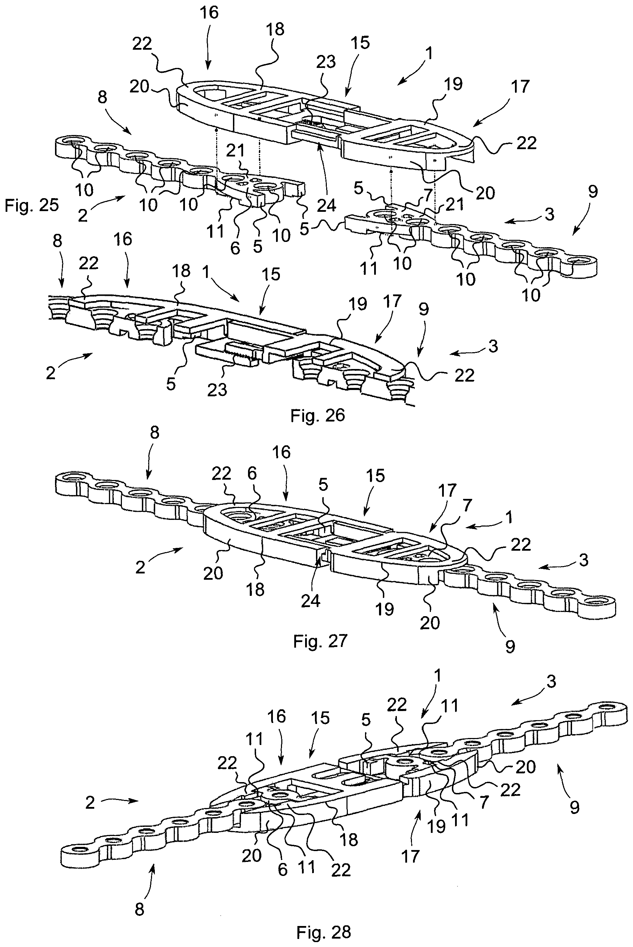

- Fig. 25: eine isometrische Darstellung eines Brustbeinverschluss-Verschließteil-Zusammenbaus in Explosionsansicht, worin der Brustbeinverschluss nach den Fig. 1 bis 4 eingesetzt ist und in dem Zustand befindlich ist, in dem das Entnahmeteil entfernt ist (Fig. 2 und 4), und mit einem zweiteiligen Verschließteil gemäß einer weiteren Ausführungsform, wobei die Teile des Verschließteils in Längsrichtung des Brustbeinverschlusses zueinander verschiebbar angeordnet sind, und wobei das Verschließteil mit Vorsprüngen versehen ist, die in die Aussparungen an Anbringbereichen der Befestigungsabschnitte einschiebbar sind,
- Fig. 26: eine isometrische Detailansicht einer Hälfte des Brustbeinverschluss-Verschließteil-Zusammenbaus nach Fig. 25, wobei das Verschließteil in einem der Befestigungsabschnitte angebrachten Zustand dargestellt ist, in dem die Vorsprünge in die Aussparungen eingeschoben sind und die Teile des Verschließteils mittels integrierter Verrasterungen zueinander gesichert sind,
- Fig. 27: eine isometrische Darstellung des gesamten Brustbeinverschluss-Verschließteil-Zusammenbaus nach Fig. 25, wobei das Verschließteil in dem in Fig. 26 dargestellten Zustand befindlich ist, und die Vorderseite des Brustbeinverschlusses gezeigt ist,
- Fig. 28: eine isometrische Darstellung der Rückseite des gesamten Brustbeinverschluss-Verschließteil-Zusammenbaus nach Fig. 26 und 27,

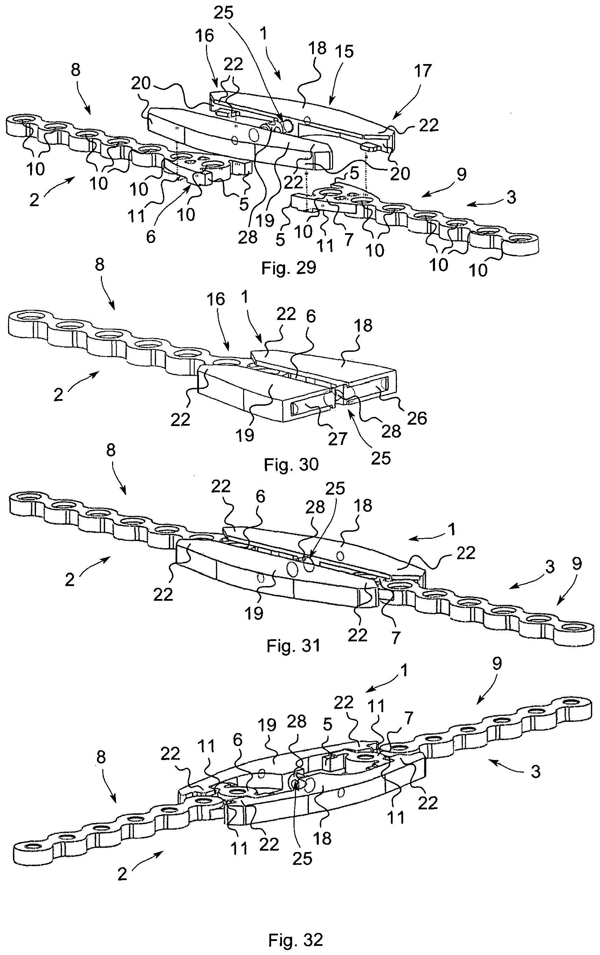

- Fig. 29: eine isometrische Darstellung eines Brustbeinverschluss-Verschließteil-Zusammenbaus in Explosionsansicht, worin der Brustbeinverschluss nach den Fig. 1 bis 4 eingesetzt ist und in dem Zustand befindlich ist, in dem das Entnahmeteil entfernt ist (Fig. 2 und 4), und mit einem zweiteiligen Verschließteil gemäß einer weiteren Ausführungsform, wobei die Teile des Verschließteils in Querrichtung des Brustbeinverschlusses zueinander verschiebbar angeordnet sind, und wobei das Verschließteil mit Vorsprüngen versehen ist, die in die Aussparungen an Anbringbereichen der Befestigungsabschnitte einschiebbar sind,
- Fig. 30: eine isometrische Detailansicht einer Hälfte des Brustbeinverschluss-Verschließteil-Zusammenbaus nach Fig. 29, wobei das Verschließteil in einem an einem der Befestigungsabschnitte angebrachten Zustand dargestellt ist, in dem die Vorsprünge in die Aussparungen eingeschoben sind und die Teile des Verschließteils mittels Spindeltrieb zueinander gesichert sind,
- Fig. 31: eine isometrische Darstellung des gesamten Brustbeinverschluss-Verschließteil-Zusammenbaus nach Fig. 29, wobei das Verschließteil in dem in Fig. 30 dargestellten Zustand befindlich ist, und die Vorderseite des Brustbeinverschlusses gezeigt ist,
- Fig. 32: eine isometrische Darstellung der Rückseite des gesamten Brustbeinverschluss-Verschließteil-Zusammenbaus nach Fig. 30 und 31,

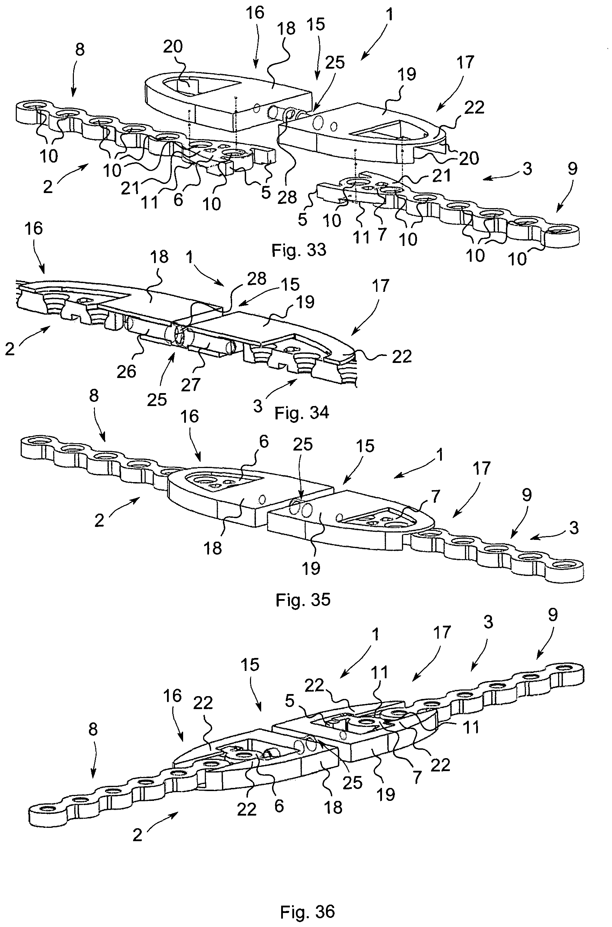

- Fig. 33: eine isometrische Darstellung eines Brustbeinverschluss-Verschließteil-Zusammenbaus in Explosionsansicht, worin der Brustbeinverschluss nach den Fig. 1 bis 4 eingesetzt ist und in dem Zustand befindlich ist, in dem das Entnahmeteil entfernt ist (Fig. 2 und 4), und mit einem zweiteiligen Verschließteil gemäß einer weiteren Ausführungsform, wobei die Teile des Verschließteils in Längsrichtung des Brustbeinverschlusses zueinander verschiebbar angeordnet sind, und wobei das Verschließteil mit Vorsprüngen versehen ist, die in die Aussparungen an Anbringbereichen der Befestigungsabschnitte einschiebbar sind,
- Fig. 34: eine isometrische Detailansicht einer Hälfte des Brustbeinverschluss-Verschließteil-Zusammenbaus nach Fig. 33, wobei das Verschließteil in einem an einem der Befestigungsabschnitte angebrachten Zustand dargestellt ist, in dem die Vorsprünge in die Aussparungen eingeschoben sind und die Teile des Verschließteils mittels integriertem Spindeltrieb zueinander gesichert sind,
- Fig. 35: eine isometrische Darstellung des gesamten Brustbeinverschluss-Verschließteil-Zusammenbaus nach Fig. 33, wobei das Verschließteil in dem in Fig. 34 dargestellten Zustand befindlich ist, und die Vorderseite des Brustbeinverschlusses gezeigt ist,
- Fig. 36: eine isometrische Darstellung der Rückseite des gesamten Brustbeinverschluss-Verschließteil-Zusammenbaus nach Fig. 34 und 35,

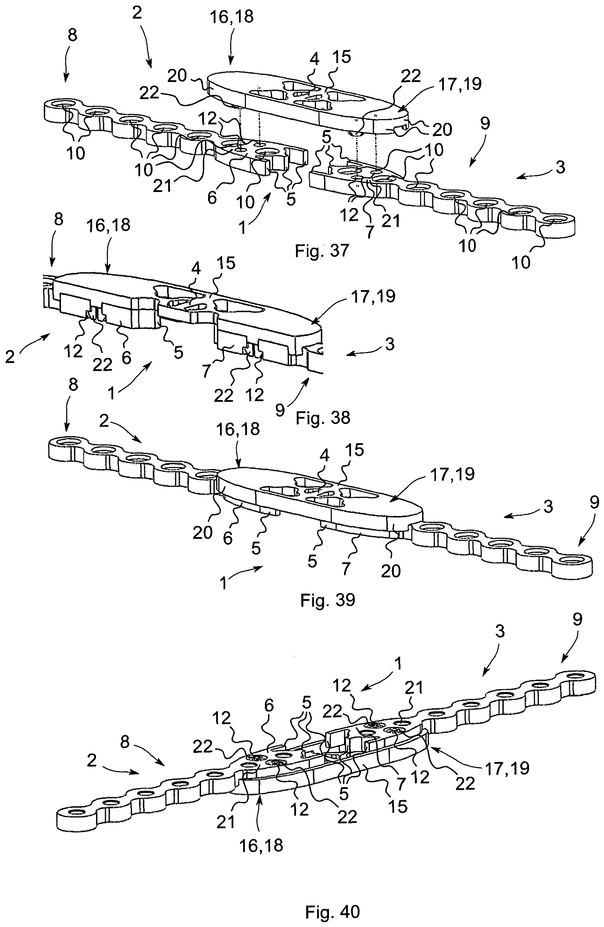

- Fig. 37: eine isometrische Darstellung eines Brustbeinverschluss-Verschließteil-Zusammenbaus in Explosionsansicht, worin der Brustbeinverschluss nach den Fig. 5 bis 8 eingesetzt ist und in dem Zustand befindlich ist, in dem das Entnahmeteil entfernt ist (Fig. 6 und 8), und mit einem einteiligen Verschließteil gemäß einer weiteren Ausführungsform, wobei das Verschließteil mit einer Schnappvorrichtung versehen ist, die in Löcher an Anbringbereichen der Befestigungsabschnitte einrastbar sind,
- Fig. 38: eine isometrische Detailansicht einer Hälfte des Brustbeinverschluss-Verschließteil-Zusammenbaus nach Fig. 37, wobei das Verschließteil in einem an einem der Befestigungsabschnitte angebrachten Zustand dargestellt ist, in dem die Schnappvorrichtung in den Löchern der Befestigungsabschnitte eingerastet ist,
- Fig. 39: eine isometrische Darstellung des gesamten Brustbeinverschluss-Verschließteil-Zusammenbaus nach Fig. 37, wobei das Verschließteil in dem in Fig. 38 dargestellten Zustand befindlich ist, und die Vorderseite des Brustbeinverschlusses gezeigt ist,
- Fig. 40: eine isometrische Darstellung der Rückseite des gesamten Brustbeinverschluss-Verschließteil-Zusammenbaus nach Fig. 38 und 39,

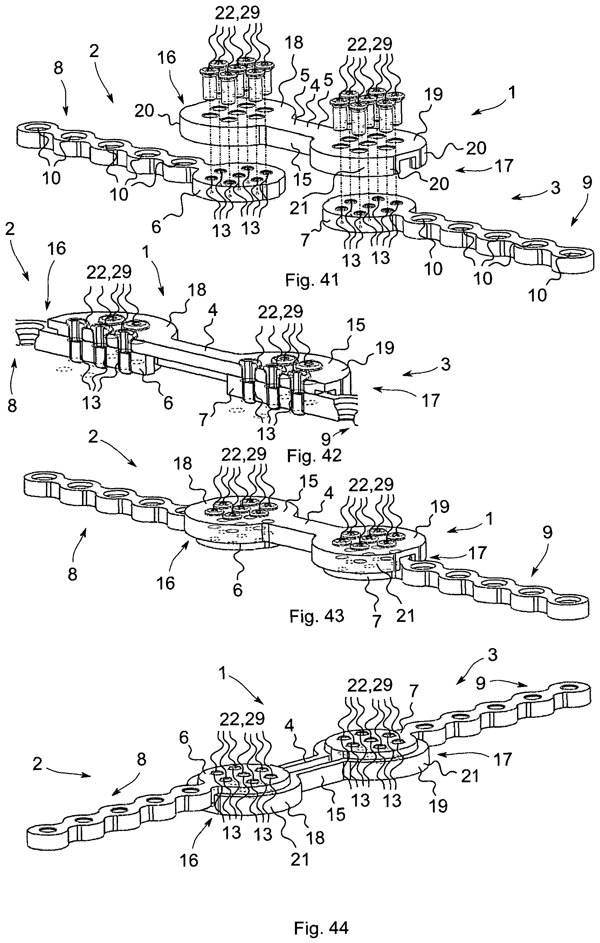

- Fig. 41: eine isometrische Darstellung eines Brustbeinverschluss-Verschließteil-Zusammenbaus in Explosionsansicht, worin der Brustbeinverschluss nach den Fig. 9 bis 12 eingesetzt ist und in dem Zustand befindlich ist, in dem das Entnahmeteil entfernt ist (Fig. 10 und 12), und mit einem einteiligen Verschließteil gemäß einer weiteren Ausführungsform, wobei das Verschließteil mit Befestigungsmitteln versehen ist, die in Löcher an Anbringbereichen der Befestigungsabschnitte befestigbar sind,
- Fig. 42: eine isometrische Detailansicht einer Hälfte des Brustbeinverschluss-Verschließteil-Zusammenbaus nach Fig. 41, wobei das Verschließteil in einem an einem der Befestigungsabschnitte angebrachten Zustand dargestellt ist, in dem die Befestigungsmittel in den Löchern der Befestigungsabschnitte befestigt sind,
- Fig. 43: eine isometrische Darstellung des gesamten Brustbeinverschluss-Verschließteil-Zusammenbaus nach Fig. 41, wobei das Verschließteil in dem in Fig. 42 dargestellten Zustand befindlich ist, und die Vorderseite des Brustbeinverschlusses gezeigt ist, und
- Fig. 44: eine isometrische Darstellung der Rückseite des gesamten Brustbeinverschluss-Verschließteil-Zusammenbaus nach Fig. 42 und 43.

Die Figuren sind lediglich schematischer Natur und dienen nur dem Verständnis der Erfindung. Dieselben Elemente sind mit denselben Bezugszeichen versehen.

In den Figuren 1 bis 20 sind verschiedene Ausführungsformen eines Brustbeinverschlusses 1 dargestellt. Jeder dieser Brustbeinverschlüsse 1 dient zum Überbrücken eines Spaltes in einem menschlichen oder tierischen Brustbein, insbesondere zum Überbrücken eines in Längsrichtung des Brustbeins/ Sternums verlaufenden Spaltes. Der Brustbeinverschluss 1 umfasst einen ersten Befestigungsabschnitt 2 zum Anbringen (an einem Knochen- und/oder Knorpelgewebeabschnitt) auf der einen Seite des Spaltes und einem zweiten Befestigungsabschnitt 3 zum Anbringen auf der anderen Seite des Spaltes. Neben diesen Befestigungsabschnitten 2 und 3 weist der Brustbeinverschluss 1 ein, im befestigten Zustand/ Betriebszustand des Brustbeinverschlusses 1 den Spalt von dem ersten Befestigungsabschnitt 2 zu dem zweiten Befestigungsabschnitt 3 überbrückendes und stofflich mit diesem verbundenes Entnahmeteil 4 auf, wobei das Entnahmeteil 4 an zumindest zwei voneinander beabstandeten Durchtrennungsstellen 5 mit jedem der Befestigungsabschnitte 2 und 3 verbunden ist und an diesen Stellen durchtrennbar ist. Das von beiden Befestigungsabschnitten 2 und 3 somit entfernbare Entnahmeteil 4 ist so bemessen, dass eine für einen Werkzeugeinsatz am Brustbein ausreichend große Arbeitsöffnung zwischen dem ersten Befestigungsabschnitt 2 und dem zweiten Befestigungsabschnitt 3 erreichbar und vorgesehen ist. Der Brustbeinverschluss 1 weist eine längliche, flache und somit plattenförmige Erstreckung auf.

Wie bspw. in Fig. 1 gut zu erkennen ist, weist der erste Befestigungsabschnitt 2 an der dem zweiten Befestigungsabschnitt 3 zugewandten Seite einen Verdickungsbereich auf, der sich im Wesentlichen quer zur Längsrichtung vergrößert und, wie nachfolgend näher erläutert, für die Aufnahme eines Verschließteils 15 vorgesehen ist. Der Verdickungsbereich des ersten Befestigungsabschnitts 2 ist nachfolgend als erster Anbringbereich 6 bezeichnet. Auch der zweite Befestigungsabschnitt 3 weist an der dem ersten Befestigungsabschnitt 2 zugewandten Seite einen Verdickungsbereich auf, der sich im Wesentlichen quer zur Längsrichtung vergrößert und, wie nachfolgend näher erläutert, für die Aufnahme eines Verschließteils 15 vorgesehen ist. Der Verdickungsbereich des zweiten Befestigungsabschnitts 3 ist nachfolgend als zweiter Anbringbereich 7 bezeichnet.

An der dem zweiten Befestigungsabschnitt 3 abgewandten Seite des ersten Anbringbereiches 6 schließt integral ein bandförmiger Grundabschnitt 8, nachfolgend als erster Grundabschnitt 8 bezeichnet, an. Dieser erste Grundabschnitt 8 ist im Wesentlichen als gelochtes Band ausgestaltet, wobei mehrere einzelne Durchgangslöcher 10 (etwa Durchgangsbohrungen oder Durchgangsausstanzungen) entlang der Längsrichtung benachbart zueinander angeordnet sind. Auch an der dem ersten Befestigungsabschnitt 2 abgewandten Seite des zweiten Anbringbereiches 6 schließt integral ein bandförmiger Grundabschnitt 9, nachfolgend als zweiter Grundabschnitt 9 bezeichnet, an, der wie der erste Grundabschnitt 8 aufgebaut ist und daher ebenfalls im Wesentlichen als gelochtes Band ausgestaltet ist und mehrere einzelne Durchgangslöcher 10 (etwa Durchgangsbohrungen oder Durchgangsausstanzungen) entlang der Längsrichtung benachbart zueinander angeordnet, aufweist.

Auch die beiden Anbringbereiche 6 und 7 weisen jeweils zumindest ein Durchgangsloch 10 (als Durchgangsbohrung ausgeführt) auf. Jede der Durchgangslöcher 10 ist dafür vorgesehen, dass es zur Befestigung an einem Knochen und/oder einem Knorpelgewebe eines Tieres oder eines Menschen mit einer Knochenschraube zusammenwirkt, wobei der Gewindeabschnitt jeder dieser Schrauben durch eines der Durchgangslöcher 10 durchsteckbar ist und der an diesen Gewindeabschnitt anschließende Schraubenkopf im befestigten Zustand der Knochenschraube den jeweiligen Befestigungsabschnitt 2, 3 gegen das Brustbein drückt. Als Alternative zu dieser Befestigung über eine Verschraubung wäre auch ein Locking-Verschluss zwischen dem Schraubenkopf und dem jeweiligen ersten oder zweiten Befestigungsabschnitt 2, 3 möglich.

Wie aus der Fig. 1 in Verbindung mit der Fig. 2 weiterhin hervorgeht, weist der in dem Ausführungsbeispiel nach den Fig. 1 bis 4 dargestellte Brustbeinverschluss 1 vier Durchtrennungsstellen 5 auf (zwei Durchtrennungsstellen 5 je Befestigungsabschnitt 2, 3). Jene, die Durchtrennungsstellen 5 aufweisenden Abschnitte des ersten und zweiten Befestigungsabschnittes 2, 3, sind stegförmig ausgestaltet, weshalb das Entnahmeteil 4 über je zwei Stege mit dem jeweiligen Anbringbereich 6, 7 verbunden ist.

Der Brustbeinverschluss 1 ist als ein integrales Bauteil ausgeformt, das stofflich (d.h. stoffschlüssig) zusammengehalten ist. Dieser integrale Brustbeinverschluss 1 ist für die erste Anbringung an einem das erste Mal getrennten Brustbein (d.h. für einen Erstverschluss des Brustbeins) geeignet. Auch ist der erste Befestigungsabschnitt 2 im Wesentlichen gleich/ spiegelverkehrt/ symmetrisch zu dem zweiten Befestigungsabschnitt 3 ausgestaltet.

Muss das Brustbein nach Befestigung des Brustbeinverschlusses 1 am Brustbein mittels den Knochenschrauben erneut geöffnet werden, etwa bei einem auftretendem Notfall, ist der Brüstbeinverschluss 1 rasch wieder zu öffnen. In einer solchen Situation dienen dann die für eine schnelle Durchtrennung des Brustbeinverschlusses 1 und Trennung der Befestigungsabschnitte 2, 3 voneinander die Durchtrennungsstellen 5. In Fig. 2 ist der Brustbeinverschluss 1 nach dem Durchtrennen der Durchtrennungsstellen 5 und einer anschließenden Entnahme des Entnahmeteils 4 dargestellt. Bspw. ist das Entnahmeteil 4 herausgeschnitten oder herausgestanzt worden.

Im Weiteren, ist in den Fig. 3 und 4 ersichtlich, dass auf der dem Brustbein im Betriebszustand zugewandten Rückseite/Unterseite des Brustbeinverschlusses 1, an jedem der Anbringbereiche 6, 7 Aussparungen 11 vorgesehen sind, deren Aufbau und Wirkungsweise später in Verbindung mit den Fig. 21 bis 36 erläutert ist.

Das zweite Ausführungsbeispiel des Brustbeinverschlusses 1 ist dann in den Fig. 5 bis 8 veranschaulicht. Bei diesem zweiten Ausführungsbeispiel sind die ersten und zweiten Befestigungsabschnitte 2, 3 im Wesentlichen wie die ersten und zweiten Befestigungsabschnitte 2, 3 der Ausführung nach den Fig. 1 bis 4 ausgeführt, weshalb deren grundsätzlicher Aufbau auch für dieses Ausführungsbeispiel zutrifft.

Im Gegensatz zu dem Brustbeinverschluss 1 des ersten Ausführungsbeispiels (Fig. 1 bis 4) unterscheidet sich der Brustbeinverschluss 1 nach Fig. 5 bis 8 in erster Linie durch die Ausgestaltung des Entnahmeteils 4, insbesondere an dem Verbindungsbereich des Entnahmeteils 4 mit den ersten und zweiten Anbringbereichen 6, 7, d. h. im Bereich der Durchtrennungsstellen 5. Das Entnahmeteil 4 bildet eine Art Fachwerkstruktur aus, über die es mit dem ersten Befestigungsabschnitt 2 einerseits und dem zweiten Befestigungsabschnitt 3 andererseits verbunden ist. Anstatt jeweils über zwei Stege, ist das Entnahmeteil 4 nun über mehrere kleine Stege, bspw. drei Stege pro Befestigungsabschnitt 2, 3 mit den ersten und zweiten Anbringbereichen 6, 7 verbunden. Daher sind auch insgesamt mindestens sechs kleinere Durchtrennungsstellen 5 zwischen den Anbringbereichen 6, 7 und dem Entnahmeteil 4 vorhanden, an denen das Entnahmeteil 4 mittels eines Trennwerkzeuges abtrennbar ist. Im Weiteren weist der Brustbeinverschluss 1 nach Fig. 5 bis 8 im Bereich der ersten und zweiten Anbringbereiche 6, 7 auch keine Aussparungen 11 auf, sondern ist in Längsrichtung/ über seine Länge hinweg gleich bleibend dick (Dicke = Erstreckung senkrecht zur Längs- und Querrichtung; Querrichtung entspricht der Richtung entlang der Längsrichtung des Brustbeins/Sternums) ausgestaltet.

Zur Aufnahme eines Verschließteils 15 dienen in den ersten und zweiten Anbringbereichen 6, 7 eingebrachte Schnappverschlussaufnahmen 12, deren näherer Aufbau und Wirkungsweise später in Verbindung mit den Fig. 37 bis 40 erläutert ist.

Ein weiteres (drittes) Ausführungsbeispiel des Brustbeinverschlusses 1 ist in den Fig. 9 bis 12 veranschaulicht. Bei diesem Ausführungsbeispiel sind die ersten und zweiten Befestigungsabschnitte 2, 3 im Wesentlichen wie die ersten und zweiten Befestigungsabschnitte 2, 3 der Ausführung nach den Fig. 1 bis 4 ausgeführt, weshalb deren grundsätzlicher Aufbau auch für dieses Ausführungsbeispiel zutrifft.

Im Gegensatz zu dem Brustbeinverschluss 1 des ersten Ausführungsbeispiels (Fig. 1 bis 4) unterscheidet sich der Brustbeinverschluss 1 nach Fig. 9 bis 12 wiederum in erster Linie durch die Ausgestaltung des Entnahmeteils 4, insbesondere an dem Verbindungsbereich des Entnahmeteils 4 mit dem ersten und zweiten Anbringbereichen 6, 7, d. h. im Bereich der Durchtrennungsstellen 5. Anstatt jeweils über zwei Stege, ist das Entnahmeteil 4 nun jeweils über nur einen Steg mit den ersten und zweiten Anbringbereichen 6, 7 den ersten und zweiten Befestigungsabschnitten 2, 3 verbunden. Daher sind nun auch nur insgesamt zwei Durchtrennungsstellen 5 zwischen den Anbringbereichen 6, 7 und dem Entnahmeteil 4 vorhanden. Auch der Brustbeinverschluss 1 nach Fig. 9 bis 12 weist im Bereich der ersten und zweiten Anbringbereiche 6, 7 keine Aussparungen 11 auf, sondern ist ebenfalls in Längsrichtung über seine Länge hinweg gleich bleibend dick ausgestaltet.

Jedoch sind auch die ersten und zweiten Anbringbereichen 6, 7 wieder zur Aufnahme eines Verschließteils 15 ausgestaltet. Jeder der Anbringbereiche 6, 7 hat eine im Wesentlichen kreisrunde und/oder plattenartige Form. In die Anbringbereiche 6, 7 sind jeweils mehrere Befestigungsmittelaufnahmen 13 eingebracht, die, wie nachfolgend in Verbindung mit den Fig. 41 bis 44 näher erläutert, zur Aufnahme eines Verschließteils 15 dienen. Der nähere Aufbau und die nähere Wirkungsweise der Befestigungsmittelaufnahmen 13 sind daher dann nachfolgend in Verbindung mit den Fig. 41 bis 44 erläutert.

Noch ein weiteres (viertes) Ausführungsbeispiel des Brustbeinverschlusses 1 ist in den Fig. 13 bis 16 veranschaulicht. Bei diesem zweiten Ausführungsbeispiel sind die ersten und zweiten Befestigungsabschnitte 2, 3 im Wesentlichen wie die ersten und zweiten Befestigungsabschnitte 2, 3 der Ausführung nach den Fig. 5 bis 8 ausgeführt, weshalb deren grundsätzlicher Aufbau auch für dieses Ausführungsbeispiel zutrifft.

Im Gegensatz zu dem Brustbeinverschluss 1 des Ausführungsbeispiels der Fig. 5 bis 8 unterscheidet sich der Brustbeinverschluss 1 nach Fig. 13 bis 16 wiederum in erster Linie durch die Ausgestaltung des Entnahmeteils 4. Genauer betrachtet sind zwei stabförmige Entnahmeteile 4 nebeneinander angeordnet, wobei jedes Entnahmeteil 4 an einer Durchtrennungsstelle 5 mit dem ersten Anbringbereich 6 verbunden ist und an einer anderen Durchtrennungsstelle 5 mit dem zweiten Anbringbereich 7 verbunden ist. Somit sind zwei Stege vorgesehen (zwei voneinander beabstandete Entnahmeteil 4), die an insgesamt vier Durchtrennungsstellen 5 die Anbringbereiche 6, 7 miteinander verbinden. Im Weiteren sind die Durchtrennungsstellen 5, wie besonders gut in den Fig. 13 und 15 erkennbar, mittels Kerben 14 geometrisch erkennbar. Diese Kerben 14 bilden eine Art Sollbruchstelle aus, in die eine Zange beim Öffnen des Brustbeinverschlusses 1 eingreift, um die beiden Befestigungsabschnitte 2, 3 voneinander unter einem möglichst geringen Kraftaufwand zu trennen.

Ein weiteres (fünftes) Ausführungsbeispiel des Brustbeinverschlusses 1 ist in den Fig. 17 bis 20 veranschaulicht. Bei diesem zweiten Ausführungsbeispiel sind die ersten und zweiten Befestigungsabschnitte 2, 3 im Wesentlichen wie die ersten und zweiten Befestigungsabschnitte 2, 3 der Ausführung nach den Fig. 5 bis 8 ausgeführt, weshalb deren grundsätzlicher Aufbau auch für dieses Ausführungsbeispiel zutrifft.

Im Gegensatz zu dem Brustbeinverschluss 1 des Ausführungsbeispiels der Fig. 5 bis 8 unterscheidet sich der Brustbeinverschluss 1 nach Fig. 17 bis 20 wiederum in erster Linie durch die Ausgestaltung des Entnahmeteils 4, insbesondere dessen Ausgestaltung im Bereich der Durchtrennungsstellen 5. Anstatt jeweils über zwei Stege, ist das Entnahmeteil 4 nun jeweils über zwei zweigeteilte Stege mit den ersten und zweiten Anbringbereichen 6, 7 der ersten und zweiten Befestigungsabschnitte 2, 3 verbunden. Auch dieses Entnahmeteil 4 bildet mittels dieser Stege eine Art Fachwerkstruktur aus, über die es mit dem ersten Befestigungsabschnitt 2 einerseits und dem zweiten Befestigungsabschnitt 3 andererseits verbunden ist. Daher sind nun insgesamt acht Durchtrennungsstellen 5 zwischen den Anbringbereichen 6, 7 und dem Entnahmeteil 4 vorhanden, an denen das Entnahmeteil 4 mittels eines Trennwerkzeuges abtrennbar ist.

In den Fig. 21 bis 44 sind nun nachfolgend verschiedene Ausführungsformen eines Verschließteils 15 dargestellt. Das Verschließteil 15 ist dabei stets für die Anbringung an einem der Brustbeinverschlüsse 1 nach den Fig. 1 bis 20 vorgesehen und dient zum erneuten Verschließen des Brustbeinverschlusses 1 (welcher an einem menschlichen oder tierischen Körper im Bereich des Brustbeins zum Verschließen eines Spaltes darin anbringbar ist), nachdem zuvor das Entnahmeteil 4 entfernt worden ist. In den Fig. 21 bis 44 ist deshalb der Brustbeinverschluss 1 stets ohne Entnahmeteil 4 dargestellt. Das Verschließteil 15 hat weiterhin einen ersten Hintergriffbereich 16 und einen zweiten Hintergriffbereich 17, wobei zum einen der erste Hintergriffbereich 16 zum formschlüssigen Befestigen an dem ersten Anbringbereich 6 des Brustbeinverschlusses 1 auf der einen Seite des Spalts vorbereitet/vorgesehen ist und zum anderen der zweite Hintergriffbereich 17 zum formschlüssigen Befestigen an dem zweiten Anbringbereich 7 des Brustbeinverschlusses 1 auf der anderen Seite des Spalts vorbereitet/vorgesehen ist. Das Verschließteil 15, wie bspw. in Fig. 21 gut zu erkennen, ist stofflich von dem Brustbeinverschluss 1 getrennt ausgestaltet. Das Verschließteil 15 ist sowohl geteilt / mehrteilig / mehrstückig (Fig. 21 bis 36), etwa zweigeteilt, oder einstückig (Fig. 37 bis 44) ausgestaltbar.

In den Figuren 21 bis 24 ist zunächst ein erstes Ausführungsbeispiel eines erfindungsgemäßen Verschließteils 15 dargestellt. Das hierin dargestellte Verschließteil 15 ist zweigeteilt und weist eine erste Verschließteilhälfte 18 und eine zweite Verschließteilhälfte 19 auf. Die beiden Verschließteilhälften 18, 19 nach den Figuren 21 bis 24 sind entlang einer in Längsrichtung verlaufenden Teilungsebene voneinander getrennt und in Querrichtung dazu verschiebbar zueinander ausgestaltet. Die erste und die zweite Verschließteilhälfte 18, 19 sind jeweils im Wesentlichen halbschalenförmig ausgestaltet und erstrecken sich in Längsrichtung von dem ersten Anbringbereich 6 zu dem zweiten Anbringbereich 7 hin. In einem ersten Endbereich (in Längsrichtung betrachtet, d.h. die Seite des Verschließteils 15, die im Betriebszustand des Verschließteils 15 dem ersten Befestigungsabschnitt 2 zugewandt ist) bilden die beiden Verschließteilhälften 18, 19 jeweils einen ersten Hintergriffbereich 16 aus. Auch in einem zweiten, dem ersten Endbereich gegenüberliegenden Endbereich (d.h. zweiter Endbereich ist die Seite des Verschließteils 15, die im Betriebszustand des Verschließteils 15 dem zweiten Befestigungsabschnitt 2 zugewandt ist) bilden die beiden Verschließteilhälften 18, 19 jeweils einen zweiten Hintergriffbereich 17 aus. Die beiden Hintergriffbereiche 16, 17 sind zur Befestigung an den oben beschriebenen Brustbeinverschlüssen 1, insbesondere dem Brustbeinverschluss 1 nach den Fig. 1 bis 4, vorgesehen.

Im ersten Hintergriffbereich 16 weisen die ersten und zweiten Verschließteilhälften 18, 19 sich quer zur Längsrichtung erstreckende und aufeinander zu gerichtete Führungsflächen 20 auf. In dem ersten Hintergriffbereich 16 ist eine erste Führungsfläche 20 an der ersten Verschließteilhälfte 18 und eine zweite Führungsfläche 20 an der zweiten Verschließteilhälfte 19 angebracht. Jede der Führungsflächen 20 erstreckt sich dabei gegensinnig/komplementär zu der ihr zugewandten Außenseite des ersten Anbringbereiches 6 in einem Verjüngungsbereich 21 (Außenseite ist die Seite, die Vorder- und Rückseite miteinander verbindet). Die Außenseiten im Verjüngungsbereich 21 verlaufen quer zur Längsrichtung derart, dass der erste Anbringbereich 6 sich in diesem Verjüngungsbereich 21 zu einer dem zweiten Anbringbereich 7 abgewandten Seite hin verjüngt, wie auch besonders gut in Fig. 21 zu erkennen. Die beiden Führungsflächen 20 verlaufen entlang der jeweiligen Außenseite des ersten Anbringbereiches 6. Die beiden Führungsflächen 20 des ersten Hintergriffbereiches 16 sind daher zum Hintergreifen der Außenseite des ersten Anbringbereiches 6 vorgesehen.

An einer oberen und einer unteren Seite jeder Führungsfläche 20 schließt wiederum jeweils ein Eindringelement 22 in Form einer Nase (auch als Rippe, Dorn, Vorsprung, Grat oder Haken bezeichenbar) an. Die Eindringelemente 22 sind integraler Bestandteil des Verschließteils 15. Die Eindringelemente 22 erstrecken sich jeweils entlang der Vorderseite und der Rückseite und liegen vorzugsweise im Betriebszustand mit ihren Innenseiten an der Vorderseite und der Rückseite des ersten Anbringbereiches 6 an. Zum formschlüssigen Zusammenwirken des ersten Anbringbereiches 6 mit dem ersten Hintergriffbereich 16, sind in der Rückseite des ersten Anbringbereiches 6 weiterhin zur Aufnahme der beiden an ihr anliegenden Eindringelemente 22 (eine oder zwei) Aussparungen 11 (auch als Ausnehmung oder Einbuchtung bezeichenbar) vorgesehen. Jede dieser Aussparungen 11 stellt eine Anschlags- und Führungsfläche in Längsrichtung zur Verfügung, an denen das jeweilige Eindringelement 22 im Betriebszustand in Anlage ist (wie in Fig. 22 bis 24 zu erkennen) und wiederum den ersten Anbringbereich 6 hintergreift. Der erste Hintergriffbereich 16 wird daher durch die Führungsflächen 20 sowie die Eindringelemente 22 gebildet. Der erste Anbringbereich 6 ist dadurch in einem befestigten Zustand/ einem Betriebszustand des Verschließteils 15 fest und formschlüssig mit dem ersten Hintergriffbereich 16 verbunden.

Der zweite Hintergriffbereich 17 ist wie der erste Hintergriffbereich 16 aufgebaut, vorzugsweise symmetrisch zu diesem ausgestaltet. Auch dieser weist folglich wiederum je Verschließteilhälfte 18, 19 eine Führungsfläche 20 sowie zwei an jede dieser Führungsflächen 20 anschließende Eindringelemente 22, wie Nasen (auch als Rippe, Dorn, Vorsprung, Grat oder Haken bezeichenbar) auf. Auch die Rückseite des zweiten Anbringbereiches 7 weist eine Aussparung 11 auf, die von den Nasen hintergriffen werden und somit in Zusammenwirken mit den Führungsflächen 20 zumindest im Betriebszustand den zweiten Anbringbereich 7 mit dem Verschließteil 15 formschlüssig verbinden. Der zweite Hintergriffbereich 17 wird daher ebenfalls durch die Führungsflächen 20 sowie die Eindringelemente 22 gebildet.

Die Anschlags- und Führungsflächen der Aussparungen 11 sind weiterhin derart schräg sowohl zur Längsrichtung als auch zu der dazu um 90° versetzten Querrichtung des Brustbeinverschlusses 1 verlaufend angeordnet, dass es bei einem Zusammenschieben der Verschließteilhälften 18, 19 in Querrichtung (da die Eindringelemente 22 an den Anschlags- und Führungsflächen der Aussparungen 11 anliegen) zu einem Aufeinanderzubewegen der Befestigungsabschnitte 2 und 3 kommt. Beim Schließen des Verschließteils 15 umgreifen die Führungsfläche 20 sowie die Eindringelemente 22 des Verschließteils 15 die Befestigungsabschnitte 2 und 3 so, dass die Befestigungsabschnitte 2 und 3 sich aufeinander zu bewegen. Somit wird der zuvor entstandene Spalt im Brustbein geschlossen. Die Verschlussmöglichkeit schließt somit eine variierende Spaltbreite.

In anderen Worten ausgedrückt sind die beiden Verschließteilhälften 18, 19, quer zur Längsrichtung, zueinander verschiebbar ausgestaltet und bilden an dem ersten und dem zweiten Endbereich (d.h. im Bereich des ersten und des zweiten Anbringbereiches 6, 7) eine Art Zangenstruktur aus, die in ihrer Spreizung veränderbar ist und den jeweiligen Anbringbereich 6, 7 über die Führungsflächen 20 festhält. Zur Fixierung in einem eingeschobenen Zustand des Verschließteils 15 in den Anbringbereichen 6, 7 (Nasen und Führungsflächen 20 hintergreifen die Aussparung und Außenseite) dient eine in Querrichtung wirkende Verrasterung 23, wobei ein erster, Verrastvorsprünge aufweisender Abschnitt der Verrasterung 23 stofflich mit der ersten Verschließteilhälfte 18 verbunden ist und ein zweiter, Gegenverrastvorsprünge aufweisender Abschnitt der Verrasterung 23 stofflich mit der zweiten Verschließteilhälfte 19 verbunden ist. Im befestigten Zustand sind die Verrastvorsprünge und die Gegenrastvorsprünge ineinander verrastet und somit die beiden Verschließteilhälften 18, 19 formschlüssig zueinander fixiert.

Weiterhin ist an dem Verschließteil 15, nämlich an der ersten Verschließteilhälfte 18, alternativ dazu aber auch zusätzlich oder stattdessen an der zweiten Verschließteilhälfte 19, ein Trennungsbereich 30 (Cut Point) vorgesehen sein, an dem das Verschließteil 15 in einem erneuten Notfall rasch wieder geöffnet werden kann. Wie besonders gut in Fig. 21 zu erkennen, ist der Trennungsbereich 30 auf dem ersten Verrastvorsprünge aufweisenden Abschnitt der Verrasterung 23 aufgebracht, sodass der jeweilige Abschnitt von der weiteren ersten Verschließteilhälfte 18 getrennt werden kann und sich dann die beiden Verschließteilhälften 18, 19 wieder auseinander ziehen lassen und von dem Brustbeinverschluss 1 entfernen lassen. Ein weiteres Ausführungsbeispiel des Verschließteils 15 ist in den Figuren 25 bis 26 dargestellt, wobei dieses Ausführungsbeispiel ähnlich zu dem zuvor beschriebenen ersten Ausführungsbeispiel nach Fig. 21 bis 24 ist. Jedoch ist dieses Verschließteil 15 nicht längs geteilt, sondern quer, d.h. entlang einer Ebene, die normal zur Längsrichtung ausgeführt ist. Der erste Hintergriffbereich 16 ist folglich nur durch eine einzelne Verschließteilhälfte, nämlich die erste Verschließteilhälfte 18 ausgebildet. Der zweite Hintergriffbereich 16 ist durch die zweite Verschließteilhälfte 19 ausgebildet. Jeder Hintergriffbereich 16, 17 ist wiederum mit zwei Führungsflächen 20 und je Führungsfläche 20 mit zwei Eindringelementen 22 ausgestattet, deren Aufbau und Erstreckung den Führungsflächen 20 und Eindringelementen 22 der Hintergriffbereiche 16, 17 der Ausführung nach den Fig. 21 bis 24 entspricht. Die Hintergriffbereiche 16 und 17 wirken im Betriebszustand mit dem ersten und zweiten Anbringbereich 6 und 7 des Brustbeinverschlusses 1 auf gleiche Weise zusammen wie die des Verschließteils 15 nach den Fig. 21 bis 24.

Zudem sind die beiden Verschließteilhälften 18, 19 über in Längsrichtung verlaufendes Führungsschienensystem/Schienensystem 24 zueinander geführt. Mittels einer Verrasterung 23 sind die beiden Verschließteilhälften 18, 19 wiederum mit den Anbringbereichen 6 und 7 verspannbar. Die Verrasterung 23 wirkt dabei nicht in Querrichtung, sondern in Längsrichtung. Ein erster, Verrastvorsprünge aufweisender Abschnitt der Verrasterung 23 ist dabei stofflich mit der ersten Verschließteilhälfte 18 verbunden und ein zweiter, Gegenverrastvorsprünge aufweisender Abschnitt der Verrasterung 23 stofflich mit der zweiten Verschließteilhälfte 19. Im befestigten Zustand sind die Verrastvorsprünge und die Gegenrastvorsprünge ineinander verrastet und somit die beiden Verschließteilhälften 18, 19 formschlüssig zueinander fixiert.

Gemäß einer weiteren Ausführungsform des Verschließteils 15, wie sie in den Fig. 29 bis 32 dargestellt ist, kann die Verrasterung 23 auch durch einen Spindeltrieb 25 ersetzt werden. Der sonstige Aufbau des Verschließteils 15 entspricht der Verschließteilausführung nach den Fig. 21 bis 24. Als das die beiden Verschließteilhälften 18 und 19 im Betriebszustand mit den Anbringbereichen 6, 7 verspannende Element dient nun der Spindelantrieb 25, der besonders gut in Fig. 30 zu erkennen ist. Der Spindelantrieb 25 weist einen ersten Spindelstangenabschnitt/eine erste Spindelstange 26 auf, die wiederum ein erstes Außengewinde aufweist, das in (einem Innengewinde) der ersten Verschließteilhälfte 18 eingeschraubt ist. Neben dem ersten Spindelstangenabschnitt 26 weist der Spindelantrieb 25 einen zweiten Spindelstangenabschnitt/ eine zweite Spindelstange 27 auf, auf der ein zweites Außengewinde angebracht ist und die wiederum in (einem Innengewinde) der zweiten Verschließteilhälfte 19 eingeschraubt ist. Beide Spindelstangenabschnitte 26, 27 (jeweils auch als Gewindestangenabschnitte/ Gewindestangen bezeichenbar) sind derart an den Verschließteilhälften 18, 19 angeordnet und relativ zueinander positioniert, dass sie koaxial zueinander verlaufend ausgerichtet sind. Zudem ist das erste Außengewinde gegensinnig zum zweiten Außengewinde ausgestaltet. D.h., dass, wenn das erste Außengewinde ein Rechtsgewinde ist, das zweite Außengewinde ein Linksgewinde ist; oder wenn das erste Außengewinde ein Linksgewinde ist, dann ist das zweite Außengewinde ein Rechtsgewinde. An ihren aufeinander zugewandten Endbereichen sind die beiden Spindelstangenabschnitte 26, 27 drehfest miteinander verbunden, bspw. stofflich miteinander verbunden. Im Verbindungsbereich zwischen den Spindelstangenabschnitten 26, 27 weist der Spindeltrieb 25 einen Betätigungsabschnitt 28 auf, der ebenfalls stofflich mit dem ersten und dem zweiten Spindelstangenabschnitt 26, 27 verbunden ist. Der Betätigungsabschnitt 28 ist dafür vorgesehen eine den Spindelantrieb 25 antreibende Betätigungskraft in die Spindelstangenabschnitte 26 und 27 einzuleiten. Der Betätigungsabschnitt 28 weist weise eine Werkzeugaufnahmenstruktur, wie Löcher in der Außenumfangsfläche, auf, an denen ein Werkzeug formschlüssig angesetzt werden kann, um den Betätigungsabschnitt 28 und somit die Spindelstangenabschnitte 26, 27 (relativ zu den Verschließteilhälften 18, 19) zu verdrehen. Bei einem Verdrehen des Betätigungsabschnittes 28 kommt es aufgrund der Aufnahme der Spindelstangenabschnitte 26, 27 in den Verschließteilhälften 18, 19 zu einem Verschieben der Verschließteilhälften 18, 19 aufeinander zu bzw. voneinander weg. Als Alternative zu der vorherigen Spindeltriebausführung kann der Betätigungsabschnitt 28 auch als separates Bauteil in Form einer Gewindehülse ausgeführt sein und stofflich von den beiden Spindelstangenabschnitten 26, 27 getrennt sein. Auch die Spindelstangenabschnitte 26, 27 wären dann getrennt voneinander/als separate Bauteile ausgeführt, wobei der erste Spindelstangenabschnitt 26 dann nicht mehr verdrehbar in der ersten Verschließteilhälfte 18 aufgenommen wäre, sondern drehfest gehalten wäre und auch der zweite Spindelstangenabschnitt 27 wäre dann nicht mehr verdrehbar in der zweiten Verschließteilhälfte 18 aufgenommen, sondern drehfest darin gehalten. An ihren aufeinander zugewandten Endbereichen wäre die Gewindehülse dann auf die Spindelstangenabschnitte 26, 27 aufgeschraubt, wobei die Gewindehülse je Spindelstangenabschnitt 26, 27 mit einem Innengewinde versehen ist, das in ein Außengewinde des jeweiligen Spindelstangenabschnittes 26, 27 eingreift. Vorzugsweise sind das erste und das zweite Außengewinde nur hinsichtlich ihrer Drehrichtung unterschiedlich ausgestaltet, hinsichtlich des Durchmesser und der Flankensteigung gleich ausgebildet, sodass die Gewindehülse dann einen gleich bleibenden Innendurchmesser aufweisen könnte. Die Gewindehülse wirkt dabei derart mit den Spindelstangenabschnitten 26 und 27 zusammen, dass bei Drehung der Gewindehülse in eine erste Drehrichtung wiederum beide Spindelstangenabschnitte 26 und 27 und somit die beiden Verschließteilhälften 18 und 19 aufeinander zu bewegt werden, bei Drehung der Gewindehülse 28 in einer zweiten Drehrichtung, die der ersten Drehrichtung entgegengerichtet ist, beide Verschließteilhälften 18 und 19 voneinander weg bewegt werden. Dadurch ist der Abstand zwischen den Verschließteilhälften 18 und 19 besonders einfach einstellbar.

Ein weiteres Ausführungsbeispiel des Verschließteils 15 ist in den Fig. 33 bis 36 dargestellt, dessen Aufbau im Wesentlichen dem Aufbau des Verschließteils 15 nach den Fig. 25 bis 28 entspricht. Dabei ist die Verrasterung 23 durch den Spindeltrieb 25 ersetzt. Lediglich die Orientierung des Spindeltriebs 25 hat sich geändert. Der Spindeltrieb 25 ist nun mit seiner Längsrichtung parallel zur Längsrichtung des Brustbeinverschlusses 1 (im Betriebszustand) ausgerichtet und nicht mehr in Querrichtung. Die Verankerung der Spindelstangenabschnitte 26 und 27 an den jeweiligen Verschließteilhälften 18 und 19 und die übrige Ausgestaltung des Spindeltriebes 25 entspricht der bereits erläuterten Ausführung nach den Fig. 29 bis 32.

Ein weiteres Ausführungsbeispiel des Verschließteils 15 ist in den Fig. 37 bis 40 dargestellt, dessen Aufbau im Wesentlichen dem Aufbau des Verschließteils 15 nach den Fig. 21 bis 24 entspricht. Die erste und die zweite Verschließteilhälfte 18, 19 sind jedoch hierin integral miteinander ausgeführt und stofflich miteinander verbunden. Die erste Verschließteilhälfte 18 ist zu der zweiten Verschließteilhälfte 19 unverschieblich. Die Verschließteilhälften 18, 19 weisen wiederum eine halbschalenartige Form auf, sind jedoch auf anderer Weise an dem ersten und zweiten Anbringbereich 6, 7 des Brustbeinverschlusses 1, insbesondere des Brustbeinverschlusses 1 nach den Fig. 5 bis 8, befestigbar. Der erste Hintergriffbereich 16, der an der ersten Verschließteilhälfte 18 angeordnet ist, weist zwei Eindringelement 22 auf. Auch der zweite Hintergriffbereich 17, der an der zweiten Verschließteilhälfte 19 angeordnet ist, weist zwei Eindringelemente 22 auf. Jedes der Eindringelemente 22 ist als zapfenförmiger Vorsprung/ Zapfen ausgestaltet und stofflich mit den Verschließteilhälften 18, 19 verbunden. Dieser Zapfen weist einen ringförmigen Vorsprungsbereich auf, der in einer als Loch/Bohrung ausgestalteten Schnappverschlussaufnahme 12 des jeweiligen Anbringbereiches 6, 7 unter Ausbildung einer Schnappverbindung/ eines Schnappverschlusses einrastbar ist. Die Zapfen sind im Wesentlichen senkrecht verlaufend zum Brustbein ausgerichtet.

Ein weiteres Ausführungsbeispiel des Verschließteils 15 ist in den Fig. 41 bis 44 dargestellt, dessen Aufbau dem Aufbau des Verschließteils 15 nach den Fig. 37 bis 40 ähnelt. Auch hierin sind die erste und die zweite Verschließteilhälfte 18, 19 integral als ein Teil ausgebildet und stofflich miteinander verbunden. Das Verschließteil 15 ist jedoch so ausgestaltet um den Brustbeinverschluss 1 nach den Fig. 9 bis 12 nach Entnahme des Entnahmeteils 4 wieder zu verschließen. Jede Verschließteilhälfte 18 und 19 weist daher eine runde Schalenform auf, in dessen Inneren der kreisrunde erste oder zweite Anbringbereich 6, 7 unter Anlage der Außenseite und der Vorderseite/Oberseite des ersten/zweiten Anbringbereiches 6, 7 an der Innenseite/Innenfläche der ersten/zweiten Verschließteilhälfte 18, 19.

Im Weiteren sind die Eindringelemente 22 nicht als Verrasterungszapfen, sondern als Befestigungsmittel 29, nämlich als Schraube, alternativ auch Bolzen oder Niet, ausgestaltet, die stofflich von den Verschließteilhälften 18, 19 getrennt sind. Jedes Befestigungsmittel 29 wirkt mit einer Befestigungsmittelaufnahme 13 in dem ersten oder zweiten Anbringbereich 6, 7 zusammen. Zur Halterung eines Befestigungsmittels 29, wie besonders gut aus Fig. 42 hervorgeht, weist die erste und die zweite Verschließteilhälfte 18, 19 ein Durchgangsloch, vorzugsweise eine Durchgangsbohrung, auf, durch das ein Befestigungsabschnitt (etwa ein Gewindeabschnitt bei einer Schraube) im Betriebszustand des Verschließteils 15 (d.h. dem Zustand, in dem das Verschließteil fest mit dem zuvor getrennten Brustbeinverschluss 1 verbunden ist) hindurch gesteckt ist/ hindurchragt. Auf der dem Brustbeinverschluss 1 abgewandten Seite/der Vorder-/Oberseite des Verschließteils 15 liegt ein Kopf (etwa ein Schraubenkopf bei einer Schraube) fest an, wodurch das Befestigungsmittel 29 zu einer Seite hin abgestützt ist. Mit einem durch das Verschließteil 15/ die jeweilige Verschließteilhälfte 18, 19 hindurchragenden Bereich (etwa des Gewindeabschnittes bei der Schraube) ist das Befestigungsmittel 29 fest in dem ersten oder dem zweiten Anbringbereich 6, 7 in der Befestigungsmittelaufnahme 13 (etwa einem Innengewinde bei der Schraube) gehalten/fixiert. Diese Eindringelemente 22 bilden daher wieder den ersten und zweiten Hintergriffbereich 16, 17 aus. Je Hintergriffbereich 16, 17 sind mehrere, vorzugsweise zwischen zwei und zehn, weiter bevorzugt zwischen fünf und acht, besonders bevorzugt sieben Befestigungsmittel 29 vorgesehen, die jeweils einem Durchgangsloch und einer Befestigungsmittelaufnahme 13 zugeordnet sind und mit diesen zusammenwirken.

Auch wenn nicht ausführlicher erläutert, sind die Brustbeinverschlüsse 1 der Ausführungen nach den Fig. 16 bis 20 dafür vorgesehen mit einem der Verschließteile 15 zusammenzuwirken und bei entnommenen Entnahmeteilen 4 durch dieses Verschließteil 15 fest verbunden zu sein. Vorzugsweise weisen auch diese Brustbeinverschlüsse 1 zumindest Aufnahmen zur formschlüssigen und/oder kraftschlüssigen Halterung der Eindringelemente 22 auf (etwa die Aussparungen 11, die Schnappverschlussaufnahmen 12 oder die Befestigungsmittelaufnahmen 13).

Weiterhin bildet ein Brustbeinverschluss 1 (mit oder ohne Entnahmeteil 4) und ein mit ihm zusammenwirkendes Verschließteil 15 ein erfindungsgemäßes Brustbeinverschlusssystem, wobei, wie bereits beschrieben, in einer Verschließstellung des Verschließteiles 15, zur festen Verbindung des ersten mit dem zweiten Befestigungsabschnitt 2, 3, der erste Hintergriffbereich 16 des Verschließteils 15 formschlüssig in dem fest mit dem ersten Befestigungsabschnitt 2, 3 verbundenen ersten Anbringbereich 6 eingreift und der zweite Hintergriffbereich 17 des Verschließteils 15 formschlüssig in einem fest mit dem zweiten Befestigungsabschnitt 3 verbundenen zweiten Anbringbereich 7 eingreift.

Wie weiterhin, insbesondere in Zusammenhang mit den Fig. 37 bis 44 erkennbar, weisen die Verschließteile 15 jeweils auch wiederum eine Trennvorrichtung nach Art eines Entnahmeteils 4 auf, das die Hintergriffbereiche 16, 17 miteinander verbindet und welches Entnahmeteil 4 im Wesentlichen wie das Entnahmeteil 4 des Brustbeinverschlusses 1 aufgebaut ist und wirkt. Das Verschließteil 15 ist daher ebenfalls, bei einem erneut auftretenden Notfall durch ein Trennwerkzeug trennbar, ohne zunächst die Eindringelemente 22 von den Anbringbereichen 6, 7 zu lösen.

Zum Verschließen eines Spaltes in einem menschlichen oder tierischen Brustbein mit einem Brustbeinverschluss 1, wird einer der Brustbeinverschlüsse 1 nach oben genannter Ausführung an einem Knochen- und/oder Knorpelgewebe angebracht (etwa angeschraubt). Der Brustbeinverschluss 1, wie er bspw. in Fig. 1 dargestellt ist, wird dabei mit seiner Rückseite/Unterseite auf fest mit dem Brustbein verbundenen Knochen oder Knorpelgewebe aufgelegt, wobei der erste Befestigungsabschnitt 2 auf einer ersten Seite (etwa der linken Seite) des Spaltes aufgelegt wird, und der zweite Befestigungsabschnitt 3 auf einer zweiten Seite (etwa der rechten Seite) des Spaltes aufgelegt wird. Der erste Anbringbereich 6 wird dabei direkt mittels Knochenschrauben (der Übersichtlichkeit halber nicht dargestellt) auf der ersten Seite des Spaltes an dem Brustbein befestigt. Der erste Grundabschnitt 8 wird dann bspw. mittels weiterer Knochenschrauben, ebenfalls auf der ersten Seite befestigt, etwa am Sternum oder an Rippen des Brustkorbs. Der zweite Anbringbereich 7 wird dabei direkt mittels Knochenschrauben (der Übersichtlichkeit halber nicht dargestellt) auf der zweiten Seite des Spaltes an dem Brustbein befestigt. Der zweite Grundabschnitt 9 wird dann bspw. mittels weiterer Knochenschrauben, ebenfalls auf der zweiten Seite befestigt, etwa am Brustkorb/ Rippen oder Sternum des Brustkorbs.

Weiterhin wird dann zum Herstellen einer Arbeitsöffnung für eine Brustbeindurchtrennung oder eine Brustbeinöffnung an einem menschlichen oder tierischen Brustbein der an einem Knochen- und/oder Knorpelgewebe angebrachte Brustbeinverschluss 1 nach einer der oben genannten Ausführungen an den Durchtrennungsstellen 5 durchtrennt und gleichzeitig das Entnahmeteil 4 von den Befestigungsabschnitten 2, 3 entfernt wird (s. bspw. Fig. 1 in Verbindung mit Fig. 2).

Zum Wiederverschließen des Brustbeinverschlusses 1 wird dann eines der Verschließteile 15 nach zumindest einer der oben genannten Ausführungen formschlüssig sowohl mit dem ersten Befestigungsabschnitt 2 als auch mit dem zweiten Befestigungsabschnitt 3 verbunden, wie es bspw. in Verbindung mit den Fig. 21 bis 23 offenbart ist. Vorzugsweise wird das Verschließteil 15 hierbei über den jeweiligen Anbringbereich 6, 7 mit dem jeweiligen Befestigungsabschnitt 2, 3 verbunden.

Im Weiteren ist das Verschließteil 15 und oder der Brustbeinverschluss 1 ganz oder teilweise aus Metall, vorzugsweise einem Titanwerkstoff/ einer Titanlegierung, besonders bevorzugt aus einem Formgedächtnismetall, oder weiter bevorzugt aus einem Kunststoff, etwa PEEK oder PEKK, hergestellt.

### Bezugszeichenliste

- 1: Brustbeinverschluss
- 2: erster Befestigungsabschnitt
- 3: zweiter Befestigungsabschnitt
- 4: Entnahmeteil
- 5: Durchtrennungsstelle
- 6: erster Anbringbereich
- 7: zweiter Anbringbereich
- 8: erster Grundabschnitt
- 9: zweiter Grundabschnitt
- 10: Durchgangsloch
- 11: Aussparung
- 12: Schnappverschlussaufnahme
- 13: Befestigungsmittelaufnahme
- 14: Kerbe
- 15: Verschließteil
- 16: erster Hintergriffbereich
- 17: zweiter Hintergriffbereich
- 18: erste Verschließteilhälfte
- 19: zweite Verschließteilhälfte
- 20: Führungsfläche
- 21: Verjüngungsbereich
- 22: Eindringelement
- 23: Verrasterung
- 24: Schienensystem
- 25: Spindeltrieb
- 26: erster Spindelstangenabschnitt
- 27: zweiter Spindelstangenabschnitt
- 28: Betätigungsabschnitt
- 29: Befestigungsmittel
- 30: Trennungsbereich

## Patentansprüche

1. Verschließteil (15) für einen Brustbeinverschluss (1), welcher an einem menschlichen oder tierischen Körper im Bereich des Brustbeins zum Verschließen eines Spaltes darin anbringbar ist, wobei das Verschließteil (15) einen ersten Hintergriffbereich (16) und einen zweiten Hintergriffbereich (17) aufweist, sowie zum einen der erste Hintergriffbereich (16) zum formschlüssigen Befestigen an einem ersten Anbringbereich (6) des Brustbeinverschlusses (1) auf der einen Seite des Spalts vorbereitet ist und zum anderen der zweite Hintergriffbereich (17) zum formschlüssigen Befestigen an einem zweiten Anbringbereich (7) des Brustbeinverschlusses (1) auf der anderen Seite des Spalts vorbereitet ist, und wobei ein als erste Verschließteilhälfte (18) ausgebildeter Bestandteil und ein als zweite Verschließteilhälfte (19) ausgebildeter Bestandteil miteinander lösbar verbunden sind, **dadurch gekennzeichnet, dass** in einem ersten Endbereich die beiden Verschließteilhälften (18, 19) jeweils den ersten Hintergriffbereich (16) ausbilden, in einem zweiten, dem ersten Endbereich gegenüberliegenden Endbereich die beiden Verschließteilhälften (18, 19) jeweils den zweiten Hintergriffbereich (17) ausbilden, und eine die lösbare Verbindung zwischen den Verschließteilhälften (18, 19) gewährende Verrasterung (23) quer zu einer Längsrichtung des Verschließteils (15) wirkend ausgebildet ist.

2. Verschließteil (15) nach Anspruch 1, **dadurch gekennzeichnet, dass** das Verschließteil (15) stofflich vom Brustbeinverschluss (1) getrennt ist.

3. Verschließteil (15) nach Anspruch 1 oder 2, **dadurch gekennzeichnet, dass** der erste und/oder der zweite Hintergriffbereich (16, 17) als Eindringelement (22), etwa als Zapfen, Schraube, Haken, Bolzen oder Dorn, ausgebildet ist und/oder eine Führungsfläche (20) ausformt.

4. Verschließteil (15) nach Anspruch 3, **dadurch gekennzeichnet, dass** das Eindringelement (22) integraler Bestandteil des Verschließteils (15) ist oder als stofflich separates Bauteil ausgeführt ist und/oder das Eindringelement (22) als Teil eines Schnappverschlusses ausgebildet ist.

5. Verschließteil (15) nach Anspruch 4, **dadurch gekennzeichnet, dass** der Schnappverschluss, zusätzlich zu einem Formschluss an der gleichen oder einer anderen Stelle, eingesetzt ist.

6. Verschließteil (15) nach einem der Ansprüche 3 bis 5, **dadurch gekennzeichnet, dass** die Führungsfläche (20) quer zur Längsrichtung des Brustbeinverschlusses (1) und/oder quer zur Längsrichtung des Spalts ausgerichtet ist.

7. Brustbeinverschlusssystem umfassend zwei voneinander beabstandete und am menschlichen oder tierischen Körper beiderseits eines Spaltes im Bereich eines Brustbeins anbringbare erste und zweite Befestigungsabschnitte (2, 3) sowie ein Verschließteil (15) nach einem der Ansprüche 1 bis 6.

8. Brustbeinverschlusssystem nach Anspruch 7, **dadurch gekennzeichnet, dass** in einer Verschließstellung des Verschließteiles (15), zur festen Verbindung des ersten mit dem zweiten Befestigungsabschnitt (2, 3), der erste Hintergriffbereich (16) des Verschließteils (15) formschlüssig in einem fest mit dem ersten Befestigungsabschnitt (2, 3) verbundenen ersten Anbringbereich (6) eingreift und der zweite Hintergriffbereich (17) des Verschließteils (15) formschlüssig in einem fest mit dem zweiten Befestigungsabschnitt (3) verbundenen zweiten Anbringbereich (7) eingreift.

## Claims

1. A closing member (15) for a sternal closure (1) adapted to be arranged on a human or animal body in the area of the sternum for closing a cleft therein, wherein the closing member (15) includes a first rear engaging area (16) and a second rear engaging area (17), wherein, on the one hand, the first rear engaging area (16) is prepared for positive securing on a first mounting area (6) of the sternal closure (1) on the one side of the cleft and, on the other hand, the second rear engaging area (17) is prepared for positive securing on a second mounting area (7) of the sternal closure (1) on the other side of the cleft, and wherein a component formed as first closing member half (18) and a component formed as second closing member half (19) are detachably connected to each other, **characterized in that** in a first end portion, each of the first and second closing member halves (18, 19) is forming the first rear engaging area (16) and in a second end portion that is arranged opposite to the first end portion each of the first and second closing member halves (18, 19) is forming the second rear engaging area (17), and a latching (23) allowing the detachable connection between the closing member halves (18, 19) is configured to act transversely to a longitudinal direction of the closing member (15).

2. The closing member (15) according to claim 1, **characterized in that** the closing member (15) is materially separated from the sternal closure (1).

3. The closing member (15) according to claim 1 or 2, **characterized in that** the first and/or second rear engaging area (16, 17) is in the form of a penetrating element (22), e.g. a journal, screw, hook, bolt or mandrel, and/or forms a guide surface (20).

4. The closing member (15) according to claim 3, **characterized in that** the penetrating element (22) is an integral part of the closing member (15) or is a materially separate component and/or the penetrating element (22) is part of a snap-fit.

5. The closing member (15) according to claim 4, **characterized in that** the snap-fit is inserted, in addition to a form-fit at the same or a different position.

6. The closing member (15) according to one of the claims 3 to 5, **characterized in that** the guide surface (20) is orientated transversely to the longitudinal direction of the sternal closure (1) and/or transversely to the longitudinal direction of the cleft.

7. A sternal closure system comprising two first and second securing portions (2, 3) spaced apart from each other and adapted to be arranged on the human or animal body on both sides of a cleft in the area of a sternum as well as a closing member (15) according to one of the claims 1 to 6.

8. The sternal closure system according to claim 7, **characterized in that** in a closing position of the closing member (15) for tight connection of the first and second securing portions (2, 3) the first rear engaging area (16) of the closing member (15) positively engages in a first mounting area (6) tightly connected to the first securing portion (2, 3) and the second rear engaging area (17) of the closing member (15) positively engages in a second mounting area (7) tightly connected to the second securing portion (3).

## Revendications

1. Pièce de fermeture (15) pour une fermeture du sternum (1) qui peut être mise en place sur un corps humain ou animal dans la zone du sternum pour la fermeture d'une fente dans celui-ci, dans laquelle la pièce de fermeture (15) présente une première zone de retenue arrière (16) et une deuxième zone de retenue arrière (17), et d'une part la première zone de retenue arrière (16) est préparée pour la fixation par coopération de formes sur une première zone de mise en place (6) de la fermeture du sternum (1) sur un côté de la fente et d'autre part la deuxième zone de retenue arrière (17) est préparée pour la fixation par coopération de formes sur une deuxième zone de mise en place (7) de la fermeture du sternum (1) sur l'autre côté de la fente, et dans laquelle un composant formé en tant que première moitié de la pièce de fermeture (18) et un composant formé en tant que deuxième moitié de la pièce de fermeture (19) sont reliés de façon séparable, **caractérisée en ce que**, dans une première zone terminale, les deux moitiés de la pièce de fermeture (18, 19) forment respectivement la première zone de retenue arrière (16), dans une deuxième zone terminale opposée à la première zone terminale, les deux moitiés de la pièce de fermeture (18, 19) forment respectivement la deuxième zone de retenue arrière (17) et un système d'enfichage (23) assurant la liaison séparable entre les moitiés de la pièce de fermeture (18, 19) est formé de façon opérationnelle, transversalement à une direction longitudinale de la pièce de fermeture (15).

2. Pièce de fermeture (15) selon la revendication 1, **caractérisée en ce que** la pièce de fermeture (15) est séparée matériellement de la fermeture du sternum (1).

3. Pièce de fermeture (15) selon la revendication 1 ou 2, **caractérisée en ce que** la première et/ou la deuxième zone de retenue arrière (16, 17) est formée en tant qu'élément de pénétration (22), à peu près comme des tenons, des vis, des crochets, des boulons ou des broches et/ou constitue une surface de guidage (20).

4. Pièce de fermeture (15) selon la revendication 3, **caractérisée en ce que** l'élément de pénétration (22) fait partie intégrante de la pièce de fermeture (15) ou est réalisé en tant qu'élément matériellement séparé et/ou l'élément de pénétration (22) est formé en tant que partie d'une fermeture à cliquet.

5. Pièce de fermeture (15) selon la revendication 4, **caractérisée en ce que** la fermeture à cliquet est utilisée en plus d'une coopération de formes sur le même endroit ou à un autre endroit.

6. Pièce de fermeture (15) selon l'une des revendications 3 à 5, **caractérisée en ce que** la surface de guidage (20) est orientée transversalement à la direction longitudinale de la fermeture du sternum (1) et/ou transversalement à la direction longitudinale de la fente.

7. Système de fermeture du sternum comprenant deux premier et deuxième segments de fixation (2, 3) à distance l'un de l'autre et pouvant être mis en place sur le corps humain ou animal de part et d'autre d'une fente dans la zone d'un sternum, ainsi qu'une pièce de fermeture (15) selon l'une des revendications 1 à 6.

8. Système de fermeture du sternum selon la revendication 7, **caractérisé en ce que**, dans une position de fermeture de la pièce de fermeture (15), pour la liaison fixe du premier segment de fixation avec le deuxième (2, 3), la première zone de retenue arrière (16) de la pièce de fermeture (15) est en prise par coopération de formes dans une première zone de mise en place (6) reliée fixement au premier segment de fixation (2, 3) et la deuxième zone de retenue arrière (17) de la pièce de fermeture (15) est en prise par coopération de formes dans une deuxième zone de mise en place (7) reliée fixement au deuxième segment de fixation (3).
